(19) Europäisches Patentamt
European Patent Office
Office européen des brevets

(11) EP 3 845 265 A1

(12) EUROPEAN PATENT APPLICATION
published in accordance with Art. 153(4) EPC

(43) Date of publication:
07.07.2021 Bulletin 2021/27

(21) Application number: 19855609.4

(22) Date of filing: 19.08.2019

(51) Int Cl.:
*A61M 25/10* (2013.01) *C08C 19/28* (2006.01)
*C08K 5/39* (2006.01) *C08L 15/00* (2006.01)

(86) International application number:
PCT/JP2019/032273

(87) International publication number:
WO 2020/045145 (05.03.2020 Gazette 2020/10)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
KH MA MD TN

(30) Priority: 29.08.2018 JP 2018160653

(71) Applicant: Zeon Corporation
Tokyo 100-8246 (JP)

(72) Inventors:
- TOMARI, Kohei
Tokyo 100-8246 (JP)
- KATO, Shinji
Tokyo 100-8246 (JP)
- HAYASHI, Misa
Tokyo 100-8246 (JP)

(74) Representative: Ehlich, Eva Susanne
Maiwald Patentanwalts- und Rechtsanwaltsgesellschaft mbH
Elisenhof
Elisenstraße 3
80335 München (DE)

# (54) PRODUCTION METHOD FOR MEDICAL BALLOON

(57) [Object] Provided is a production method for a medical balloon that is capable of suppressing the occurrence of the symptom of delayed-type allergy (Type IV) in addition to immediate-type allergy (Type I).

[Solving Means] The production method for a medical balloon, includes: a step of molding a composition containing a carboxy-modified conjugated diene polymer, a vulcanizing agent, and a xanthogen compound into the shape of a balloon. The step of molding the composition into the shape of a balloon includes a dip molding step that is a step of dipping a balloon-shaped mold in the composition in the form of latex.

Figure 1

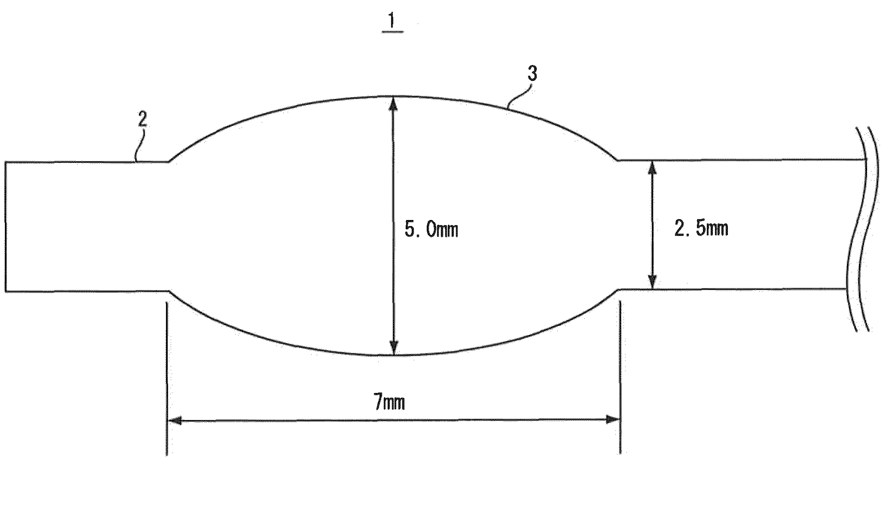


EP 3 845 265 A1

Printed by Jouve, 75001 PARIS (FR)

## Description

### TECHNICAL FIELD

The present invention relates to a production method for a medical balloon.

### BACKGROUND ART

For example, balloon-equipped medical devices such as a balloon catheter and a balloon-equipped endoscope have been used in various treatments or examinations. As a medical balloon that is used to configure balloon-equipped medical devices, a medical balloon obtained by performing dip molding with respect to natural rubber latex has been widely used. However, the natural rubber latex contains protein that causes the symptom of immediate-type allergy (Type I) in the human body, and thus, may have a problem as a balloon that is directly in contact with the human body. For this reason, as the material of the medical balloon, it has been considered that synthetic rubber latex is used instead of the natural rubber latex.

For example, in Patent Document 1, a latex composition in which zinc oxide, sulfur, and a vulcanization promoter are compounded with synthetic polyisoprene latex that is synthetic rubber is disclosed as a composition for dip molding that can also be used in the production of a balloon. However, in the case of using the latex composition that is specifically disclosed in Patent Document 1, the occurrence of the immediate-type allergy (Type I) due to protein derived from natural rubber can be prevented, but there is a concern that an allergy symptom of delayed-type allergy (Type IV) occurs due to the vulcanization promoter that is contained in the balloon as a dip molding body.

### CITATION LIST

### PATENT DOCUMENT

Patent Document 1: WO 2014/129547 A1

### SUMMARY OF THE INVENTION

### PROBLEM TO BE SOLVED BY THE INVENTION

The invention has been made in consideration of such circumstances, and an object thereof is to provide a production method for a medical balloon that is capable of suppressing the occurrence of the symptom of not only immediate-type allergy (Type I) but also delayed-type allergy (Type IV).

### MEANS FOR SOLVING PROBLEM

As result of intensive studies of the present inventors for attaining the object described above, it has been found that the object described above can be attained by molding a medical balloon with a composition containing a carboxy-modified conjugated diene polymer, a vulcanizing agent, and a xanthogen compound, and the invention has been completed.

That is, according to the invention, a production method for a medical balloon, including: a step of molding a composition containing a carboxy-modified conjugated diene polymer, a vulcanizing agent, and a xanthogen compound into the shape of a balloon, is provided.

In the invention, the vulcanizing agent is capable of including a sulfur-based vulcanizing agent.

In the invention, the composition may further contain a metal oxide or a typical metal compound other than an oxide, as an activating agent.

In the invention, it is preferable that the step of molding the composition into the shape of a balloon includes a dip molding step that is a step of dipping a balloon-shaped mold in the composition in the form of latex.

A medical balloon that is obtained by a production method for a medical balloon according to the invention is capable of suppressing the occurrence of the symptom of not only immediate-type allergy (Type I) but also delayed-type allergy (Type IV).

### BRIEF DESCRIPTION OF DRAWINGS

Fig. 1 is a diagram illustrating a mold used in the molding (the production) of a medical balloon in Examples of the invention.

MODE FOR CARRYING OUT THE INVENTION

A production method for a medical balloon according to the invention, includes: a step of molding a composition containing a carboxy-modified conjugated diene polymer, a vulcanizing agent, and a xanthogen compound into the shape of a balloon. The invention is not particularly limited, but it is preferable to mold a target medical balloon by applying a dip molding step that is a step of obtaining the composition to be the material of the medical balloon, as a latex composition in a form in which at least the carboxy-modified conjugated diene polymer of the components in the composition is dispersed in water as a dispersion medium, and of dipping a balloon-shaped mold in the composition. Hereinafter, an embodiment of the invention will be described in detail.

[Composition to Be Material of Medical Balloon]

The carboxy-modified conjugated diene polymer that is contained in the composition to be the material of the medical balloon is a conjugated diene polymer having a carboxyl group in a molecular structure. The carboxy-modified conjugated diene polymer may be obtained in accordance with a known method of the related art, and for example, can be obtained by modifying a conjugated diene polymer with a carboxyl group-containing compound, or by copolymerizing a conjugated diene monomer and a carboxyl group-containing monomer.

Conjugated Diene Polymer

The conjugated diene polymer used to obtain the carboxy-modified conjugated diene polymer is not particularly limited insofar as the conjugated diene polymer is a polymer in which a main monomer unit is a unit derived from a conjugated diene monomer, and examples thereof include synthetic polyisoprene, a styrene-isoprene-styrene block copolymer (SIS), and a nitrile group-containing conjugated diene-based copolymer. Among them, synthetic polyisoprene, SIS, and the like, having an isoprene unit, are preferable, and synthetic polyisoprene is particularly preferable.

In a case where synthetic polyisoprene is used as the conjugated diene polymer, synthetic polyisoprene may be an isoprene homopolymer, or may be a copolymer of isoprene and other ethylenically unsaturated monomers copolymerizable with isoprene. The content of the isoprene unit in synthetic polyisoprene is preferably greater than or equal to 70 weight%, is more preferably greater than or equal to 90 weight%, is even more preferably greater than or equal to 95 weight%, and is particularly preferably 100 weight% (the isoprene homopolymer), with respect to the total monomer unit, from the viewpoint of easily obtaining a medical balloon that is flexible and is excellent in strength.

Examples of the other ethylenically unsaturated monomers copolymerizable with isoprene include a conjugated diene monomer other than isoprene, such as butadiene, chloroprene, and 1,3-pentadiene; an ethylenically unsaturated nitrile monomer such as acrylonitrile, methacrylonitrile, fumaronitrile, and $\alpha$-chloroacrylonitrile; a vinyl aromatic monomer such as styrene and alkyl styrene; and an ethylenically unsaturated carboxylic ester monomer such as (meth)methyl acrylate (indicating "methyl acrylate and/or methyl methacrylate", hereinafter, the same applies to ethyl (meth)acrylate and the like), ethyl (meth)acrylate, butyl (meth)acrylate, and 2-ethyl hexyl (meth)acrylate. Only one type of such other ethylenically unsaturated monomers copolymerizable with isoprene may be used, or a plurality of types thereof may be used together.

Synthetic polyisoprene can be obtained by a known method of the related art, for example, by performing solution polymerization with respect to isoprene and the other ethylenically unsaturated monomers copolymerizable with isoprene, which are used as necessary, in an inactive polymerization solvent, by using a Ziegler-based polymerization catalyst containing aluminum trialkyl-titanium tetrachloride or a alkyl lithium polymerization catalyst such as lithium n-butyl and lithium sec-butyl. A polymer solution of synthetic polyisoprene obtained by the solution polymerization may be directly used in the production of synthetic polyisoprene latex, and can also be used in the production of the synthetic polyisoprene latex by extracting solid synthetic polyisoprene from the polymer solution, and then, by dissolving the solid synthetic polyisoprene in an organic solvent. Note that, as described below, the synthetic polyisoprene latex can be used in the production of latex of the carboxy-modified conjugated diene polymer.

In a case where the polymer solution of synthetic polyisoprene is obtained by the method described above, impurities such as the residue of the polymerization catalyst remaining in the polymer solution may be removed. In addition, an age inhibitor described below may be added to the solution during the polymerization or after the polymerization. In addition, commercially available solid synthetic polyisoprene can also be used.

As the isoprene unit of synthetic polyisoprene, there are four types of a cis bonding unit, a trans bonding unit, a 1,2-vinyl bonding unit, and a 3,4-vinyl bonding unit, in accordance with a bonding state of isoprene. A content ratio of the cis bonding unit of the isoprene unit in synthetic polyisoprene is preferably greater than or equal to 70 weight%, is more preferably greater than or equal to 90 weight%, and is even more preferably greater than or equal to 95 weight%, with respect to the total isoprene unit, from the viewpoint of improving the strength of the medical balloon to be obtained.

A weight average molecular weight of synthetic polyisoprene is preferably 10,000 to 5,000,000, is more preferably 500,000 to 4,000,000, and is even more preferably 800,000 to 3,000,000, in terms of standard polystyrene of gel permeation chromatography analysis. By setting the weight average molecular weight of synthetic polyisoprene to be in the range described above, the strength of the medical balloon to be obtained is improved, and the synthetic polyisoprene latex tends to be easily produced.

In addition, a polymer Mooney viscosity ($ML_{1+4}$, 100°C) of synthetic polyisoprene is preferably 50 to 80, is more preferably 60 to 80, and is even more preferably 70 to 80.

Examples of a method for obtaining the synthetic polyisoprene latex include (1) a method for producing the synthetic polyisoprene latex by emulsifying a solution or a fine suspension liquid of synthetic polyisoprene that is dissolved or finely dispersed in an organic solvent, in water, in the presence of an anionic surfactant, and as necessary, by removing the organic solvent, and (2) a method for directly producing the synthetic polyisoprene latex by performing emulsion polymerization or suspension polymerization with respect to isoprene alone or a mixture of isoprene and an ethylenically unsaturated monomer copolymerizable with isoprene, in the presence of an anionic surfactant, and the production method of (1) described above is preferable from the viewpoint that synthetic polyisoprene having a high ratio of the cis bonding unit in the isoprene unit can be used, and a medical balloon excellent in mechanical properties such as strength is easily obtained.

Examples of the organic solvent used in the production method of (1) described above are capable of including an aromatic hydrocarbon solvent such as benzene, toluene, and xylene; an alicyclic hydrocarbon solvent such as cyclopentane, cyclopentene, cyclohexane, and cyclohexene; an aliphatic hydrocarbon solvent such as pentane, hexane, and heptane; and a halogenated hydrocarbon solvent such as methylene chloride, chloroform, and ethylene dichloride. Among them, an alicyclic hydrocarbon solvent is preferable, and cyclohexane is particularly preferable.

Note that, a use amount of the organic solvent is preferably less than or equal to 2,000 parts by mass, is more preferably 20 to 1,500 parts by mass, and is even more preferably 500 to 1500 parts by mass, with respect to 100 parts by mass of synthetic polyisoprene.

Examples of the anionic surfactant used in the production method of (1) described above include a fatty acid salt such as sodium laurate, potassium myristate, sodium palmitate, potassium oleate, sodium linolenate, and sodium rosinate; an alkyl benzene sulfonate such as sodium dodecyl benzene sulfonate, potassium dodecyl benzene sulfonate, sodium decyl benzene sulfonate, and potassium decyl benzene sulfonate; an alkyl sulfosuccinate such as sodium di(2-ethyl hexyl) sulfosuccinate, potassium di(2-ethyl hexyl) sulfosuccinate, and sodium dioctyl sulfosuccinate; an alkyl sulfate such as sodium lauryl sulfate and potassium lauryl sulfate; a polyoxyethylene alkyl ether sulfate such as sodium polyoxyethylene lauryl ether sulfate and potassium polyoxyethylene lauryl ether sulfate; and a monoalkyl phosphate such as sodium lauryl phosphate and potassium lauryl phosphate.

Among such anionic surfactants, a fatty acid salt, an alkyl benzene sulfonate, an alkyl sulfosuccinate, an alkyl sulfate, and a polyoxyethylene alkyl ether sulfate are preferable, and a fatty acid salt and an alkyl benzene sulfonate are particularly preferable.

In addition, it is preferable that at least one type selected from the group consisting of an alkyl benzene sulfonate, an alkyl sulfosuccinate, an alkyl sulfate, and a polyoxyethylene alkyl ether sulfate, and a fatty acid salt are used together, and it is particularly preferable that an alkyl benzene sulfonate and a fatty acid salt are used together, from the viewpoint that a trace amount of remaining polymerization catalyst derived from synthetic polyisoprene (in particular, aluminum and titanium) can be more efficiently removed, and the generation of an aggregate at the time of producing the latex composition is suppressed. Here, sodium rosinate and potassium rosinate are preferable as a fatty acid salt, and sodium dodecyl benzene sulfonate and potassium dodecyl benzene sulfonate are preferable as an alkyl benzene sulfonate. In addition, only one type of such surfactants may be used, or two or more types thereof may be used together.

Note that, as described above, at least one type selected from the group consisting of an alkyl benzene sulfonate, an alkyl sulfosuccinate, an alkyl sulfate, and a polyoxyethylene alkyl ether sulfate, and a fatty acid salt are used together, and thus, latex to be obtained contains at least one type selected from an alkyl benzene sulfonate, an alkyl sulfosuccinate, an alkyl sulfate, and a polyoxyethylene alkyl ether sulfate, and a fatty acid salt.

In addition, in the production method of (1) described above, a surfactant other than the anionic surfactant may be used together, and a reactive surfactant can also be used together.

Further, a non-ionic surfactant such as polyoxyethylene alkyl ether, polyoxyethylene alkyl phenol ether, polyoxyethylene alkyl ester, and polyoxyethylene sorbitan alkyl ester may be used together. However, in a case where the medical balloon is molded by dip molding, and a coagulant is used, it is necessary to perform adjustment such that coagulation is not inhibited.

A use amount of the anionic surfactant used in the production method of (1) described above is preferably 0.1 to 50 parts by mass, and is more preferably 0.5 to 30 parts by mass, with respect to 100 parts by mass of synthetic polyisoprene. Note that, in a case where two or more types of surfactants are used, it is preferable that the total use amount is set to be in the range described above. That is, for example, in a case where at least one type selected from an alkyl benzene sulfonate, an alkyl sulfosuccinate, an alkyl sulfate, and a polyoxyethylene alkyl ether sulfate, and a fatty acid salt are used together, it is preferable that the total use amount is set to be in the range described above. In

a case where the use amount of the anionic surfactant is excessively small, there is a concern that a large amount of aggregates are generated in the emulsification, and on the contrary, in a case where the use amount of the anionic surfactant is excessively large, foam is easily formed, and pinholes may occur in the medical balloon to be obtained.

In addition, in a case where at least one type selected from an alkyl benzene sulfonate, an alkyl sulfosuccinate, an alkyl sulfate, and a polyoxyethylene alkyl ether sulfate, and a fatty acid salt are used together as the anionic surfactant, a use ratio thereof is preferably in a range of 1 : 1 to 10 : 1, and is more preferably in a range of 1 : 1 to 7 : 1, in a weight ratio of "Fatty Acid Salt" : "Sum of at Least One Type of Surfactant Selected from Alkyl Benzene Sulfonate, Alkyl Sulfosuccinate, Alkyl Sulfate, and Polyoxyethylene Alkyl Ether Sulfate". In a case where the use ratio of at least one type of surfactant selected from an alkyl benzene sulfonate, an alkyl sulfosuccinate, an alkyl sulfate, and a polyoxyethylene alkyl ether sulfate is excessively large, there is a concern that foaming becomes severe at the time of handling synthetic polyisoprene, and thus, an operation such as still standing for a long period of time or the addition of an antifoaming agent is required, which may lead to degradation in workability and an increase in the cost.

The amount of water used in the production method of (1) described above is preferably 10 to 1,000 parts by mass, is more preferably 30 to 500 parts by mass, and is most preferably 50 to 200 parts by mass, with respect to 100 parts by mass of an organic solvent solution of synthetic polyisoprene. Examples of the type of water to be used include hard water, soft water, ion-exchange water, distilled water, and zeolite water, and soft water, ion-exchange water, and distilled water are preferable.

A device emulsifying the solution or the fine suspension liquid of synthetic polyisoprene that is dissolved or finely dispersed in the organic solvent, in water, in the presence of the anionic surfactant, can be used without being particularly limited insofar as the device, in general, is commercially available as an emulsification machine or a dispersion machine. A method for adding the anionic surfactant to the solution or the fine suspension liquid of the synthetic polyisoprene is not particularly limited, and the anionic surfactant may be added in advance to any one or both of water and the solution or the fine suspension liquid of synthetic polyisoprene, may be added to an emulsified liquid during an emulsification operation, or may be collectively added or dividedly added.

Examples of an emulsification device include a batch emulsification machine such as Product Name "Homogenizer" (manufactured by IKA Works GmbH & Co. KG), Product Name "Polytron" (manufactured by Kinematica AG), and Product Name "TK Auto Homo Mixer" (manufactured by Tokushu Kika Kogyo Co., Ltd.); a continuous emulsification machine such as Product Name "TK Pipeline-Homo Mixer" (manufactured by Tokushu Kika Kogyo Co., Ltd.), Product Name "Colloid Mill" (manufactured by Shinko Pantec Co.,Ltd.), Product Name "Slasher" (manufactured by NIPPON COKE & ENGINEERING CO., LTD.), Product Name "Trigonal Wet Fine Pulverizer" (manufactured by Mitsui Miike Chemical Engineering Machinery, Co., Ltd.), Product Name "Cavitron" (manufactured by EUROTEC LIMITED), Product Name "Milder" (manufactured by Pacific Machinery & Engineering Co.,Ltd), and Product Name "Fine Flow Mill" (manufactured by Pacific Machinery & Engineering Co.,Ltd); a high-pressure emulsification machine such as Product Name "Microfluidizer" (manufactured by MIZUHO INDUSTRIAL CO.,LTD.), Product Name "Nanomizer" (manufactured by NANOMIZER Inc.), and Product Name "APV Gaulin" (manufactured by Apv Gaulin, Inc.); a membrane emulsification machine such as Product Name "Membrane Emulsification Machine" (manufactured by REICA Co.,Ltd.); an oscillating emulsification machine such as Product Name "Vibro Mixer" (manufactured by REICA Co.,Ltd.); and an ultrasonic emulsification machine such as Product Name "Ultrasonic Homogenizer" (manufactured by Branson Ultrasonics Corporation). Note that, the condition of the emulsification operation using the emulsification device is not particularly limited, and a treatment temperature, a treatment time, and the like may be suitably selected such that a desired dispersion state is obtained.

In the production method of (1) described above, it is desirable to remove the organic solvent from an emulsified substance obtained through the emulsification operation.

As a method for removing the organic solvent from the emulsified substance, a method is preferable in which the content of the organic solvent (preferably the alicyclic hydrocarbon solvent) in the synthetic polyisoprene latex to be obtained can be less than or equal to 500 weight ppm, and for example, a method such as reduced-pressure distillation, atmospheric distillation, steam distillation, and centrifugal separation can be adopted.

In the method of (1) described above, it is desirable that the synthetic polyisoprene latex is obtained by removing the organic solvent from the emulsified substance obtained through the emulsification operation. The method for removing the organic solvent from the emulsified substance is not particularly limited insofar as the method is a method in which the total content of the organic solvent in the synthetic polyisoprene latex to be obtained can be less than or equal to 500 weight ppm, and a method such as reduced-pressure distillation, atmospheric distillation, steam distillation, and centrifugal separation can be adopted.

Further, as necessary, in order to increase a solid content concentration of the synthetic polyisoprene latex after the organic solvent is removed, a concentration operation may be performed by a method such as reduced-pressure distillation, atmospheric distillation, centrifugal separation, and membrane concentration, and in particular, it is preferable to perform the centrifugal separation from the viewpoint that the solid content concentration of the synthetic polyisoprene latex can be increased and the amount of residual surfactant in the synthetic polyisoprene latex can be reduced.

It is preferable that the centrifugal separation, for example, is implemented by using a continuous centrifugal separation machine in a condition in which a centrifugal force is preferably 100 to 10,000 G, the solid content concentration of the synthetic polyisoprene latex before the centrifugal separation is preferably 2 to 15 weight%, a flow rate of the synthetic polyisoprene latex to be sent to the centrifugal separation machine is preferably 500 to 1700 kg/hr, and a back pressure (a gauge pressure) of the centrifugal separation machine is preferably 0.03 to 1.6 MPa, and the synthetic polyisoprene latex can be obtained a light liquid after the centrifugal separation. Accordingly, the amount of residual surfactant in the synthetic polyisoprene latex can be reduced.

The solid content concentration of the synthetic polyisoprene latex is preferably 30 to 70 weight%, and is more preferably 40 to 70 weight%. In a case where the solid content concentration is excessively low, the solid content concentration of the latex composition to be obtained as described above decreases, and thus, a film thickness of the medical balloon to be obtained decreases and a breakage easily occurs. On the contrary, in a case where the solid content concentration is excessively high, the viscosity of the synthetic polyisoprene latex increases, and thus, transfer in pipework or stirring in a preparation tank may be difficult.

A volume average particle diameter of the synthetic polyisoprene latex is preferably 0.1 to 10 μm, is more preferably 0.5 to 3 μm, and is even more preferably 0.5 to 2.0 μm. By setting the volume average particle diameter to be in the range described above, a latex viscosity is suitable, and thus, the latex can be easily handled, and the formation of a film on the surface of the latex can be suppressed at the time of storing the synthetic polyisoprene latex.

In addition, additives such as a pH adjuster, an antifoaming agent, an antiseptic agent, a cross-linking agent, a chelate agent, an oxygen scavenger, a dispersant, and an age inhibitor may be compounded with the synthetic polyisoprene latex.

Examples of the pH adjuster include a hydroxide of an alkali metal such as sodium hydroxide and potassium hydroxide; a carbonate of an alkali metal such as sodium carbonate and potassium carbonate; a hydrogen carbonate of an alkali metal such as sodium hydrogen carbonate; ammonia; and an organic amine compound such as trimethyl amine and triethanol amine, and a hydroxide of an alkali metal or ammonia is preferable.

In addition, as described above, a styrene-isoprene-styrene block copolymer (SIS) can be used as the conjugated diene polymer. Note that, in SIS, "S" indicates a styrene block, and "I" indicates an isoprene block.

SIS can be obtained by a known method of the related art, for example, by performing block copolymerization with respect to isoprene and styrene, in an inactive polymerization solvent, by using an active organic metal such as lithium n-butyl as an initiator. Then, a polymer solution of the obtained SIS may be directly used in the production of SIS latex, and can also be used in the production of the SIS latex by extracting solid SIS from the polymer solution, and then, by dissolving the solid SIS in an organic solvent. Note that, as described below, the SIS latex can be used in the production of the latex of the carboxy-modified conjugated diene polymer. A production method of SIS latex is not particularly limited, and a method for producing the SIS latex by emulsifying a solution or a fine suspension liquid of SIS that is dissolved or finely dispersed in an organic solvent, in water, in the presence of a surfactant, and as necessary, by removing the organic solvent is preferable.

At this time, impurities such as the residue of the polymerization catalyst remaining in the polymer solution after the synthesis may be removed. In addition, an age inhibitor described below may be added during the polymerization or after the polymerization. In addition, commercially available solid SIS can also be used.

The same solvents as those in the case of producing the synthetic polyisoprene latex described above can be used as the organic solvent, an aromatic hydrocarbon solvent and an alicyclic hydrocarbon solvent are preferable, and cyclohexane and toluene are particularly preferable.

Note that, a use amount of the organic solvent is generally 50 to 2,000 parts by mass, is preferably 80 to 1,000 parts by mass, is more preferably 10 to 500 parts by mass, and is even more preferably 150 to 300 parts by mass, with respect to 100 parts by mass of SIS.

The same surfactants as those in the case of producing the synthetic polyisoprene latex described above can be exemplified as the surfactant, an anionic surfactant is preferable, and sodium rosinate and sodium dodecyl benzene sulfonate are particularly preferable.

A use amount of the surfactant is preferably 0.1 to 50 parts by mass, and is more preferably 0.5 to 30 parts by mass, with respect to 100 parts by mass of SIS. In a case where the use amount is excessively small, the stability of the latex tends to be degraded, and on the contrary, in a case where the use amount is excessively large, foam is easily formed, and a problem may occur at the time of performing molding for obtaining a medical balloon.

The amount of water used in the production method of SIS latex described above is preferably 10 to 1,000 parts by mass, is more preferably 30 to 500 parts by mass, and is most preferably 50 to 100 parts by mass, with respect to 100 parts by mass of an organic solvent solution of SIS. Examples of the type of water to be used include hard water, soft water, ion-exchange water, distilled water, and zeolite water. In addition, a polar solvent represented by alcohol such as methanol may be used together with water.

The same device as that in the case of the synthetic polyisoprene described above can be exemplified as a device emulsifying the organic solvent solution or the fine suspension liquid of SIS in water, in the presence of the

surfactant. Then, an addition method of the surfactant is not particularly limited, and the surfactant may be added in advance to any one or both of the organic solvent solution and the fine suspension liquid of SIS, may be added to an emulsified liquid during an emulsification operation, or may be collectively added or dividedly added.

In the production method of SIS latex described above, it is preferable to obtain the SIS latex by removing the organic solvent from an emulsified substance obtained through the emulsification operation. A method for removing the organic solvent from the emulsified substance is not particularly limited, and a method such as reduced-pressure distillation, atmospheric distillation, steam distillation, and centrifugal separation can be adopted.

In addition, as necessary, in order to increase a solid content concentration of the SIS latex after the organic solvent is removed, a concentration operation may be performed by a method such as reduced-pressure distillation, atmospheric distillation, centrifugal separation, and membrane concentration.

The solid content concentration of the SIS latex is preferably 30 to 70 weight%, and is more preferably 50 to 70 weight%. In a case where the solid content concentration is excessively low, the solid content concentration of the latex composition to be obtained as described above decreases, and thus, the film thickness of the medical balloon to be obtained decreases and a breakage easily occurs. On the other hand, in a case where the solid content concentration is excessively high, the viscosity of the SIS latex increases, and thus, the transfer in the pipework or the stirring in the preparation tank may be difficult.

In addition, additives such as a pH adjuster, an antifoaming agent, an antiseptic agent, a cross-linking agent, a chelate agent, an oxygen scavenger, a dispersant, and an age inhibitor may be compounded with the SIS latex. The same pH adjusters as those in the case of the synthetic polyisoprene described above can be exemplified as the pH adjuster, and a hydroxide of an alkali metal or ammonia is preferable.

The content of a styrene unit in a styrene block of SIS that is contained in the SIS latex to be obtained as described above is preferably 70 to 100 weight%, is more preferably 90 to 100 weight%, and is even more preferably 100 weight%, with respect to the total monomer unit in the styrene block.

In addition, the content of an isoprene unit in an isoprene block of SIS is preferably 70 to 100 weight%, is more preferably 90 to 100 weight%, and is even more preferably 100 weight%, with respect to the total monomer unit in the isoprene block.

Note that, a content ratio of the styrene unit to the isoprene unit in SIS is generally in a range of 1 : 99 to 90 : 10, is preferably in a range of 3 : 97 to 70 : 30, is more preferably in a range of 5 : 95 to 50 : 50, and is even more preferably in a range of 10 : 90 to 30 : 70, in a weight ratio of "Styrene Unit: Isoprene Unit".

A weight average molecular weight of SIS is preferably 10,000 to 1,000,000, is more preferably 50,000 to 500,000, and is even more preferably 100,000 to 300,000, in terms of standard polystyrene of gel permeation chromatography analysis. By setting the weight average molecular weight of SIS to be in the range described above, balance between the strength and the flexibility of the medical balloon to be obtained is improved, and the SIS latex tends to be easily produced.

A volume average particle diameter of latex particles (SIS particles) in the SIS latex is preferably 0.1 to 10 $\mu$m, is more preferably 0.5 to 3 $\mu$m, and is even more preferably 0.5 to 2.0 $\mu$m. By setting the volume average particle diameter of the latex particles to be in the range described above, the latex viscosity is suitable, and thus, the latex can be easily handled, and the formation of a film on the surface of the latex can be suppressed at the time of storing the SIS latex.

In addition, as described above, the nitrile group-containing conjugated diene-based copolymer can also be used as the conjugated diene polymer.

The nitrile group-containing conjugated diene-based copolymer is a copolymer obtained by copolymerizing an ethylenically unsaturated nitrile monomer with a conjugated diene monomer, and may be a copolymer obtained by copolymerizing other ethylenically unsaturated monomers copolymerizable with the conjugated diene monomer and the ethylenically unsaturated nitrile monomer, which are used as necessary.

Examples of the conjugated diene monomer include 1,3-butadiene, isoprene, 2,3-dimethyl-1,3-butadiene, 2-ethyl-1,3-butadiene, 1,3-pentadiene, and chloroprene. Among them, 1,3-butadiene and isoprene are preferable, and 1,3-butadiene is more preferable. Such conjugated diene monomers can be independently used, or two or more types thereof can be used by being combined. A content ratio of a conjugated diene monomer unit to be formed by the conjugated diene monomer in the nitrile group-containing conjugated diene-based copolymer is preferably 56 to 78 weight%, is more preferably 56 to 73 weight%, and is even more preferably 56 to 68 weight%. By setting the content of the conjugated diene monomer unit to be in the range described above, the medical balloon to be obtained can be more excellent in texture and stretch while having sufficient strength.

The ethylenically unsaturated nitrile monomer is not particularly limited insofar as the ethylenically unsaturated nitrile monomer is an ethylenically unsaturated monomer having a nitrile group, and examples thereof include acrylonitrile, methacrylonitrile, fumaronitrile, $\alpha$-chloroacrylonitrile, and $\alpha$-cyanoethyl acrylonitrile. Among them, acrylonitrile and methacrylonitrile are preferable, and acrylonitrile is more preferable. Such ethylenically unsaturated nitrile monomers can be independently used, or two or more types thereof can be used by being combined. A content ratio of an ethylenically

unsaturated nitrile monomer unit to be formed by the ethylenically unsaturated nitrile monomer in the nitrile group-containing conjugated diene-based copolymer is preferably 20 to 40 weight%, is more preferably 25 to 40 weight%, and is even more preferably 30 to 40 weight%. By setting the content of the ethylenically unsaturated nitrile monomer unit to be in the range described above, the medical balloon to be obtained can be more excellent in the texture and the stretch while having sufficient strength.

Examples of the other ethylenically unsaturated monomers copolymerizable with the conjugated diene monomer and the ethylenically unsaturated nitrile monomer are capable of including an ethylenically unsaturated carboxylic acid monomer that is an ethylenically unsaturated monomer having a carboxyl group; a vinyl aromatic monomer such as styrene, alkyl styrene, and vinyl naphthalene; fluoroalkyl vinyl ether such as fluoroethyl vinyl ether; an ethylenically unsaturated amide monomer such as (meth)acrylamide, N-methylol (meth)acrylamide, N,N-dimethylol (meth)acrylamide, N-methoxymethyl (meth)acrylamide, and N-butoxymethyl (meth)acrylamide; an ethylenically unsaturated carboxylic ester monomer such as (meth)methyl acrylate, ethyl (meth)acrylate, butyl (meth)acrylate, 2-ethyl hexyl (meth)acrylate, trifluoroethyl (meth)acrylate, tetrafluoropropyl (meth)acrylate, dibutyl maleate, dibutyl fumarate, diethyl maleate, methoxymethyl (meth)acrylate, ethoxyethyl (meth)acrylate, methoxyethoxyethyl (meth)acrylate, cyanomethyl (meth)acrylate, (meth)acrylate-2-cyanoethyl, (meth)acrylate-1 -cyanopropyl, (meth)acrylate-2-ethyl-6-cyanohexyl, (meth)acrylate-3-cyanopropyl, hydroxyethyl (meth)acrylate, hydroxypropyl (meth)acrylate, glycidyl (meth)acrylate, and dimethyl aminoethyl (meth)acrylate; and a cross-linkable monomer such as divinyl benzene, polyethylene glycol di(meth)acrylate, polypropylene glycol di(meth)acrylate, trimethylol propane tri(meth)acrylate, and pentaerythritol (meth)acrylate. Such ethylenically unsaturated monomers can be independently used, or two or more types thereof can be used by being combined.

The ethylenically unsaturated carboxylic acid monomer is not particularly limited insofar as the ethylenically unsaturated carboxylic acid monomer is an ethylenically unsaturated monomer having a carboxyl group, and examples thereof include an ethylenically unsaturated monocarboxylic acid monomer such as an acrylic acid and a methacrylic acid; an ethylenically unsaturated polycarboxylic acid monomer such as an itaconic acid, a maleic acid, and a fumaric acid; an ethylenically unsaturated polycarboxylic anhydride such as a maleic anhydride and a citraconic anhydride; and a partial ester monomer of an ethylenically unsaturated polycarboxylic acid, such as monobutyl fumarate, monobutyl maleate, and mono-2-hydroxypropyl maleate. Among them, an ethylenically unsaturated monocarboxylic acid is preferable, and a methacrylic acid is particularly preferable. Such ethylenically unsaturated carboxylic acid monomers can also be used as an alkali metal salt or an ammonium salt. In addition, the ethylenically unsaturated carboxylic acid monomers can be independently used, or two or more types thereof can be used by being combined. A content ratio of an ethylenically unsaturated carboxylic acid monomer unit to be formed by the ethylenically unsaturated carboxylic acid monomer in the nitrile group-containing conjugated diene-based copolymer is preferably 2 to 5 weight%, is more preferably 2 to 4.5 weight%, and is even more preferably 2.5 to 4.5 weight%. By setting the content of the ethylenically unsaturated carboxylic acid monomer unit to be in the range described above, the medical balloon to be obtained can be excellent in the texture and the stretch while having sufficient strength.

A content ratio of the other monomer units to be formed by the other ethylenically unsaturated monomers in the nitrile group-containing conjugated diene-based copolymer is preferably less than or equal to 10 weight%, is more preferably less than or equal to 5 weight%, and is even more preferably less than or equal to 3 weight%.

The nitrile group-containing conjugated diene-based copolymer can be obtained by copolymerizing a monomer mixture containing the monomers described above, and a method for performing copolymerization by emulsion polymerization is preferable. A known method of the related art can be adopted as an emulsion polymerization method.

When the monomer mixture containing the monomers described above is subjected to the emulsion polymerization, a polymerization subsidiary material that is generally used, such as an emulsifier, a polymerization initiator, and a molecular weight adjuster, can be used. An addition method of the polymerization subsidiary material is not particularly limited, and may be any method of an initial collective addition method, a divided addition method, a continuous addition method, and the like.

The emulsifier is not particularly limited, and examples thereof are capable of including a non-ionic emulsifier such as polyoxyethylene alkyl ether, polyoxyethylene alkyl phenol ether, polyoxyethylene alkyl ester, and polyoxyethylene sorbitan alkyl ester; an anionic emulsifier such as an alkyl benzene sulfonate such as potassium dodecyl benzene sulfonate and sodium dodecyl benzene sulfonate, a higher alcohol sulfate, and an alkyl sulfosuccinate; a cationic emulsifier such as alkyl trimethyl ammonium chloride, dialkyl ammonium chloride, and benzyl ammonium chloride; and a reactive emulsifier for emulsion polymerization. Among them, an anionic emulsifier is preferable, an alkyl benzene sulfonate is more preferable, and potassium dodecyl benzene sulfonate and sodium dodecyl benzene sulfonate are particularly preferable. Such emulsifiers can be independently used, or two or more types thereof can be used by being combined. A use amount of the emulsifier is preferably 0.1 to 10 parts by mass with respect to 100 parts by mass of the monomer mixture.

The polymerization initiator is not particularly limited, and examples thereof are capable of including an inorganic peroxide such as sodium persulfate, potassium persulfate, ammonium persulfate, potassium superphosphate, and hydrogen peroxide; an organic peroxide such as diisopropyl benzene hydroperoxide, cumene hydroperoxide, t-butyl hydroperoxide, 1,1,3,3-tetramethyl butyl hydroperoxide, 2,5-dimethyl hexane-2,5-dihydroperoxide, di-t-butyl peroxide, di-$\alpha$-cumyl peroxide, acetyl peroxide, isobutyryl peroxide, and benzoyl peroxide; and an azo compound such as azobisisobutyronitrile, azobis-2,4-dimethyl valeronitrile, and methyl azobisisobutyrate. Such polymerization initiators can be independently used, or two or more types thereof can be used by being combined. A use amount of the polymerization initiator is preferably 0.01 to 10 parts by mass, and is more preferably 0.01 to 2 parts by mass, with respect to 100 parts by mass of the monomer mixture.

In addition, the peroxide initiator can be used as a redox-based polymerization initiator by being combined with a reducing agent. The reducing agent is not particularly limited, and examples thereof include a compound having metal ions in a reduction state, such as ferrous sulfate and cuprous naphthenate; a sulfonate compound such as sodium methane sulfonate; and an amine compound such as dimethyl aniline. Such reducing agents can be independently used, or two or more types thereof can be used by being combined. It is preferable that a use amount of the reducing agent is 3 to 1000 parts by mass with respect to 100 parts by mass of the peroxide.

The amount of water used in the emulsion polymerization is preferably 80 to 600 parts by mass, and is particularly preferably 100 to 200 parts by mass, with respect to 100 parts by mass of all of the monomers to be used.

Examples of an addition method of the monomer include a method for collectively adding the monomers to be used into a reaction vessel, a method for continuously or intermittently adding the monomers in accordance with the progress of the polymerization, and a method for adding a part of the monomers to react at a specific inversion rate, and then, for continuously or intermittently adding the residual monomers to be polymerized, and any method may be adopted. In a case where the monomers are mixed and are continuously or intermittently added, the composition of the mixture may be constant, or may be changed. In addition, each of the monomers may be added to the reaction vessel after mixing in advance various monomers to be used, or may be separately added to the reaction vessel.

Further, as necessary, a polymerization subsidiary material such as a chelate agent, a dispersant, a pH adjuster, a deoxygenating agent, and a particle diameter adjuster can also be used, and the type and a use amount thereof are not particularly limited.

A polymerization temperature at the time of performing the emulsion polymerization is not particularly limited, and is generally 3 to 95°C, and is preferably 5 to 60°C. A polymerization time is approximately 5 to 40 hours.

As described above, the monomer mixture is subjected to the emulsion polymerization, a polymerization system is cooled or a polymerization terminator is added at a time point of reaching a predetermined polymerization inversion rate, and thus, a polymerization reaction is terminated. The polymerization inversion rate at the time of terminating the polymerization reaction is preferably greater than or equal to 90 weight%, and is more preferably greater than or equal to 93 weight%.

The polymerization terminator is not particularly limited, and examples thereof include hydroxyl amine, a hydroxyamine sulfate, diethyl hydroxyl amine, a hydroxyamine sulfonic acid and an alkali metal salt thereof, sodium dimethyl dithiocarbamate, a hydroquinone derivative, a catechol derivative, and an aromatic hydroxydithiocarboxylic acid such as a hydroxydimethyl benzene thiocarboxylic acid, a hydroxydiethyl benzene dithiocarboxylic acid, and a hydroxydibutyl benzene dithiocarboxylic acid, and an alkali metal salt thereof. A use amount of the polymerization terminator is preferably 0.05 to 2 parts by mass with respect to 100 parts by mass of the monomer mixture.

After the polymerization reaction is terminated, as desired, the unreacted monomer is removed, and a solid content concentration or pH is adjusted, and thus, the latex of the nitrile group-containing conjugated diene-based copolymer can be obtained.

In addition, as necessary, an age inhibitor, an antiseptic agent, an antibacterial agent, a dispersant, and the like may be suitably added to the latex of the nitrile group-containing conjugated diene-based copolymer.

A number average particle diameter of the latex of the nitrile group-containing conjugated diene-based copolymer is preferably 60 to 300 nm, and is more preferably 80 to 150 nm. The particle diameter can be adjusted to a desired value by a method for adjusting the use amount of the emulsifier and the polymerization initiator, or the like.

As described above, synthetic polyisoprene, a styrene-isoprene-styrene block copolymer (SIS), a nitrile group-containing conjugated diene-based copolymer, and the like can be used as the conjugated diene polymer used to obtain the carboxy-modified conjugated diene polymer, but the carboxy-modified conjugated diene polymer is not limited thereto, and a butadiene polymer, a styrene-butadiene copolymer, and the like may be used.

The butadiene polymer may be a homopolymer of 1,3-butadiene as the conjugated diene monomer, or may be a copolymer obtained by copolymerizing other ethylenically unsaturated monomers copolymerizable with 1,3-butadiene as the conjugated diene monomer.

In addition, the styrene-butadiene copolymer is a copolymer obtained by copolymerizing styrene with 1,3-butadiene as the conjugated diene monomer, or may be a copolymer obtained by copolymerizing other ethylenically unsaturated monomers copolymerizable with styrene and 1,3-butadiene, which are used as necessary.

Latex of Carboxy-Modified Conjugated Diene Polymer

In order to obtain the latex composition that can be used as the material of the medical balloon, the latex of the carboxy-modified conjugated diene polymer can be used. The carboxy-modified conjugated diene polymer configuring the latex of the carboxy-modified conjugated diene polymer can be obtained by modifying the conjugated diene polymer described above with a compound having a carboxyl group. Alternatively, in a case where a polymer having an ethylenically unsaturated carboxylic acid monomer unit is used as the conjugated diene polymer, the conjugated diene polymer is not modified with the compound having a carboxyl group, but the conjugated diene polymer can be directly used as the carboxy-modified conjugated diene polymer.

By using the latex of the carboxy-modified conjugated diene polymer in order to obtain the latex composition, the occurrence of an aggregate in the latex composition to be obtained can be suppressed, and thus, in the case of producing the medical balloon by using the latex composition, a defect rate of the balloon can be reduced. Further, the strength of the medical balloon to be formed by using the latex composition to be obtained is further improved by using the latex of the carboxy-modified conjugated diene polymer in order to obtain the latex composition.

A method for modifying the conjugated diene polymer with the compound having a carboxyl group is not particularly limited, and examples thereof include a method for graft-polymerizing the compound having a carboxyl group with respect to with respect to the conjugated diene polymer in a water phase. The method for graft-polymerizing the compound having a carboxyl group with respect to with respect to the conjugated diene polymer in the water phase is not particularly limited, a known method of the related art may be used, and for example, a method for adding the compound having a carboxyl group and a polymerization catalyst used in the graft polymerization (a graft polymerization catalyst) to the latex of the conjugated diene polymer, and then, for allowing the compound having a carboxyl group to react with the conjugated diene polymer in a water phase is preferable.

The graft polymerization catalyst is not particularly limited, and examples thereof are capable of including an inorganic peroxide such as sodium persulfate, potassium persulfate, ammonium persulfate, potassium superphosphate, and hydrogen peroxide; an organic peroxide such as diisopropyl benzene hydroperoxide, cumene hydroperoxide, t-butyl hydroperoxide, 1,1,3,3-tetramethyl butyl hydroperoxide, di-t-butyl peroxide, isobutyryl peroxide, and benzoyl peroxide; and an azo compound such as 2,2'-azobisisobutyronitrile, azobis-2,4-dimethyl valeronitrile, and methyl azobisisobutyrate, and an organic peroxide is preferable, and 1,1,3,3-tetramethyl butyl hydroperoxide is particularly preferable, from the viewpoint of further improving the strength of the medical balloon to be obtained. Only one type of such graft polymerization catalyst may be used, or two or more types thereof may be used by being combined.

A use amount of the graft polymerization catalyst is different in accordance with the type thereof, and is preferably 0.1 to 10 parts by mass, and is more preferably 0.2 to 5 parts by mass, with respect to 100 parts by mass of the conjugated diene polymer. In addition, a method for adding the graft polymerization catalyst is not particularly limited, and a known addition method such as collective addition, divided addition, and continuous addition can be adopted.

In addition, in a case where an organic peroxide is used as the graft polymerization catalyst, the organic peroxide can be used as a redox-based polymerization initiator by being combined with a reducing agent. The reducing agent is not particularly limited, and examples thereof include a compound having metal ions in a reduction state, such as ferrous sulfate and cuprous naphthenate; a sulfonate compound such as sodium methane sulfonate; and an amine compound such as dimethyl aniline. Only one type of such reducing agents may be used, or two or more types thereof may be used by being combined.

An additive amount of the organic peroxide is not particularly limited, and is preferably 0.01 to 3 parts by mass, and is more preferably 0.1 to 1 parts by mass, with respect to 100 parts by mass the conjugated diene polymer contained in the latex of the conjugated diene polymer.

An additive amount of the reducing agent is not particularly limited, and it is preferable that the additive amount is 0.01 to 1 parts by mass with respect to 1 part by mass of the organic peroxide.

An addition method of the organic peroxide and the reducing agent is not particularly limited, and each known addition method such as collective addition, divided addition, and continuous addition can be used.

When the compound having a carboxyl group reacts with the conjugated diene polymer, it is preferable that the reaction is performed in the presence of a dispersant.

The dispersant is not particularly limited, and an anionic surfactant such as a derivative of an aromatic sulfonic acid, an fatty acid salt, an alkyl benzene sulfonate, an alkyl sulfosuccinate, an alkyl sulfate, a polyoxyethylene alkyl ether sulfate, and a monoalkyl phosphate is preferable, and a derivative of an aromatic sulfonate is more preferable, as the dispersant. Note that, such dispersants can be independently used, or two or more types thereof can be used by being combined.

The derivative of the aromatic sulfonate is not particularly limited, and a compound represented by General Formula (1) described below is preferable.

[Formula 1]

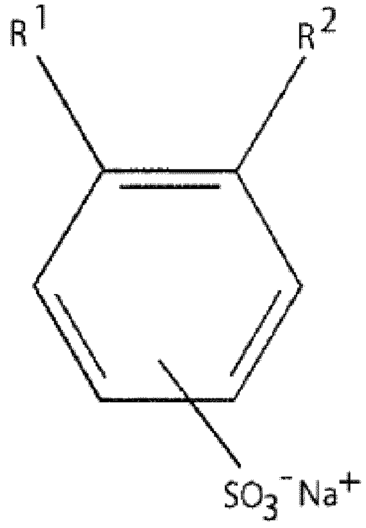

···(1)

(In General Formula (1) described above, $R^1$ and $R^2$ are each independently a hydrogen atom or an arbitrary organic group, and $R^1$ and $R^2$ may be bonded to each other to form a ring structure.)

In a case where $R^1$ and $R^2$ are not bonded to each other, an organic group that can be $R^1$ and $R^2$ is not particularly limited, and examples thereof include an alkyl group having 1 to 30 carbon atoms, such as a methyl group, an ethyl group, an n-propyl group, an isopropyl group, an n-butyl group, an isobutyl group, a sec-butyl group, a t-butyl group, an n-pentyl group, an n-hexyl group, an n-heptyl group, an n-octyl group, an n-nonyl group, and an n-decyl group; a cycloalkyl group having 3 to 30 carbon atoms, such as a cyclopropyl group, a cyclopentyl group, a cyclohexyl group, a cycloheptyl group, and a cyclooctyl group; an aryl group having 6 to 30 carbon atoms, such as a phenyl group, a biphenyl group, a naphthyl group, and an anthranil group; and a hydrocarbyl oxy group having 1 to 30 carbon atoms, such as a methoxy group, an ethoxy group, an n-butoxy group, an isobutoxy group, an n-butoxy group, an isobutoxy group, a sec-butoxy group, a t-butoxy group, an n-pentyl oxy group, an n-hexyl oxy group, and a phenoxy group. Note that, such organic groups may have a substituent, and the position of the substituent can be an arbitrary position.

In addition, in a case where $R^1$ and $R^2$ are bonded to each other to form the ring structure, the ring structure is not particularly limited, an aromatic compound is preferable, an aromatic compound having a benzene ring, such as benzene and naphthalene, is more preferable, and naphthalene is particularly preferable. Note that, such ring structures may have a substituent, and the position of the substituent can be an arbitrary position.

Among the compounds represented by General Formula (1) described above, a compound in which $R^1$ and $R^2$ are bonded to each other to form a ring structure and to form a benzene ring structure in General Formula (1) described above is particularly preferable as the derivative of the aromatic sulfonate. More specifically, it is preferable to use a compound having a structure represented by General Formula (2) described below.

[Formula 2]

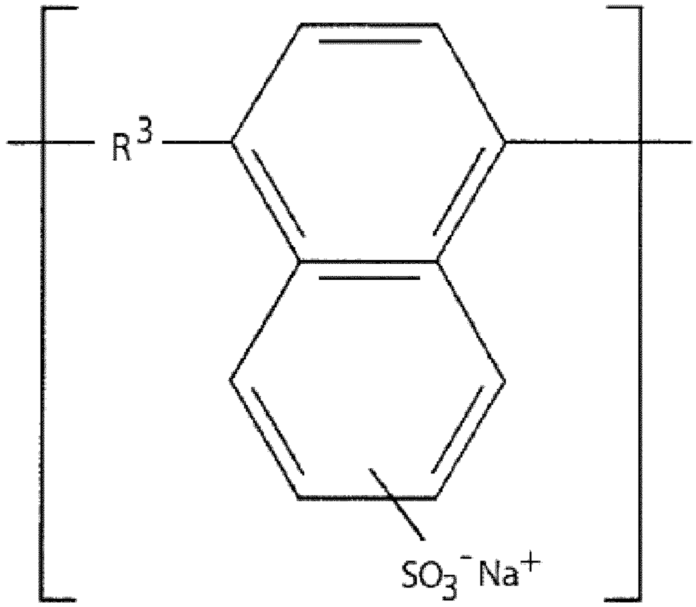

...(2)

(In General Formula (2) described above, $R^3$ is a divalent hydrocarbon group that may have a substituent.)

In General Formula (2) described above, $R^3$ is not particularly limited insofar as $R^3$ is a divalent hydrocarbon group that may have a substituent, an alkylene group having 1 to 10 carbon atoms is preferable, and a methylene group is particularly preferable.

In addition, a compound repeatedly having the structure represented by General Formula (2) described above is preferable as the derivative of the aromatic sulfonate, and the number of repeating units of the structure represented by General Formula (2) described above is not particularly limited, and is preferably 10 to 100, and is more preferably 20 to 50.

A weight average molecular weight of the derivative of the aromatic sulfonate is preferably 500 to 100,000, is more preferably 3,000 to 50,000, and is even more preferably 5,000 to 30,000.

An additive amount of the dispersant is not particularly limited, and is preferably 0.01 to 10 parts by mass, and is more preferably 0.1 to 5 parts by mass, with respect to 100 parts by mass of the conjugated diene polymer contained in the latex, from the viewpoint that the occurrence of an aggregate can be more effectively suppressed even in a case where the solid content concentration of the latex of the conjugated diene polymer is increased.

A method for adding the dispersant to the latex of the conjugated diene polymer is not particularly limited, A known addition method such as collective addition, divided addition, and continuous addition can be adopted. In addition, the dispersant may be directly added to the latex, or an aqueous solution of the dispersant may be prepared in advance, and the prepared aqueous solution of the dispersant may be added to the latex.

A reaction temperature when the compound having a carboxyl group reacts with the conjugated diene polymer is not particularly limited, and is preferably 15 to 80°C, and is more preferably 30 to 50°C. A reaction time of the reaction of the compound having a carboxyl group may be suitably set in accordance with the reaction temperature described above, and is preferably 30 to 300 minutes, and is more preferably 60 to 120 minutes.

A solid content concentration of the latex of the conjugated diene polymer in the reaction of the compound having a carboxyl group is not particularly limited, and is preferably 5 to 60 weight%, and is more preferably 10 to 40 weight%.

Examples of the compound having a carboxyl group are capable of including an ethylenically unsaturated monocarboxylic acid monomer such as an acrylic acid and a methacrylic acid; an ethylenically unsaturated polycarboxylic acid monomer such as an itaconic acid, a maleic acid, a fumaric acid, and a butene tricarboxylic acid; a partial ester monomer of an ethylenically unsaturated polycarboxylic acid, such as monobutyl fumarate, monobutyl maleate, and mono2-hydroxypropyl maleate; and a polycarboxylic anhydride such as maleic anhydride and a citraconic anhydride, an ethylenically unsaturated monocarboxylic acid monomer is preferable, and an acrylic acid and a methacrylic acid are particularly preferable. Note that, only one type of such monomers may be used, or two or more types thereof may be used together.

In addition, the carboxyl group described above is intended to include a carboxylate salt group such as an alkali metal carboxylate group, an ammonium carboxylate group, or the like.

A use amount of the compound having a carboxyl group is preferably 0.01 parts by mass to 100 parts by mass, is more preferably 0.01 parts by mass to 40 parts by mass, and is even more preferably 0.5 parts by mass to 20 parts

by mass, with respect to 100 parts by mass of the conjugated diene polymer. By setting the use amount of the compound having a carboxyl group to be in the range described above, the viscosity of the latex to be obtained is more suitable, the transfer is easily performed, and thus, the strength of the medical balloon to be formed by using the latex composition to be obtained is further improved.

A method for adding the compound having a carboxyl group to the latex is not particularly limited, and a known addition method such as collective addition, divided addition, and continuous addition can be adopted.

An inversion rate of the graft polymerization is preferably greater than or equal to 95 weight%, and is more preferably greater than or equal to 97 weight%. By setting the inversion rate of the graft polymerization to be in the range described above, the strength of the medical balloon to be obtained is further improved.

A modification rate of the compound having a carboxyl group in the carboxy-modified conjugated diene polymer is not particularly limited, and is preferably 0.01 to 10 mol%, and is more preferably 0.5 to 5 mol%. Note that, the modification rate is represented by Expression (i) described below.

$$\text{Modification Rate (Mol\%)} = (X/Y) \times 100 \cdots\text{(i)}$$

Note that, in Expression (i) described above, X indicates the number of carboxyl groups in the carboxy-modified conjugated diene polymer, Y indicates the total number of structure units of the carboxy-modified conjugated diene polymer. X can be obtained by measuring the carboxy-modified conjugated diene polymer with $^1$H-NMR In addition, Y can be obtained by calculating (Weight Average Molecular Weight (Mw) of Carboxy-Modified Conjugated Diene Polymer)/(Average Molecular Weight according to Content Ratio of Each Structure Unit Configuring Carboxy-Modified Conjugated Diene Polymer).

Additives such as a pH adjuster, an antifoaming agent, an antiseptic agent, a chelator, an oxygen scavenger, a dispersant, and an age inhibitor, which are generally compounded in the field of latex, may be compounded with the latex of the carboxy-modified conjugated diene polymer.

Examples of the pH adjuster include a hydroxide of an alkali metal such as sodium hydroxide and potassium hydroxide; a carbonate of an alkali metal such as sodium carbonate and potassium carbonate; a hydrogen carbonate of an alkali metal such as sodium hydrogen carbonate; ammonia; and an organic amine compound such as trimethyl amine and triethanol amine, and a hydroxide of an alkali metal or ammonia is preferable. Note that, the pH of the latex of the carboxy-modified conjugated diene polymer at this time is not particularly limited, and in a case where a xanthogen compound, an activating agent, and a vulcanizing agent are compounded with the latex of the carboxy-modified conjugated diene polymer to be the latex composition, and the latex composition is aged in a predetermined condition, it is preferable that the pH of the latex composition before the aging is less than 10.

In addition, as necessary, in order to increase a solid content concentration of the latex of the carboxy-modified conjugated diene polymer after the graft polymerization, a concentration operation may be performed by a method such as reduced-pressure distillation, atmospheric distillation, centrifugal separation, and membrane concentration, and it is preferable to perform the centrifugal separation from the viewpoint that the amount of residual anionic surfactant in the latex of the carboxy-modified conjugated diene polymer can be adjusted.

In a case where the latex of the carboxy-modified conjugated diene polymer is put in a centrifugal separation machine after the graft polymerization, in order to improve mechanical stability of the latex, a pH adjuster is added in advance, and it is preferable that the pH of the latex is set to be greater than or equal to 7, and it is more preferable that pH is set to be greater than or equal to 9. Note that, a carboxyl group introduced by modification is in the state of a salt, at the time of adjusting the pH of the latex.

The solid content concentration of the latex of the carboxy-modified conjugated diene polymer is preferably 30 to 70 weight%, and is more preferably 40 to 70 weight%. By setting the solid content concentration to be in the range described above, the occurrence of an aggregate in the latex can be more effectively suppressed, and the separation of the polymer particles at the time of storing the latex can be more effectively suppressed.

In addition, a content ratio of a structure unit derived from the compound having a carboxyl group in the latex of the carboxy-modified conjugated diene polymer (a content ratio of a monomer unit having a carboxyl group to be contained in a polymer by copolymerization) is preferably 0.01 to 50 weight%, is more preferably 0.5 to 40 weight%, is even more preferably 1 to 30 weight%, and is particularly preferably 1 to 15 weight%, with respect to the total monomer unit. By setting the content ratio of the structure unit derived from the compound having a carboxyl group to be in the range described above, the mechanical stability of the latex composition to be obtained is further improved, and the flexibility and the strength of the medical balloon to be formed by using the latex composition to be obtained are further improved.

Xanthogen Compound

The latex composition that can be used as the material of the medical balloon contains a xanthogen compound, in addition to the latex of the carboxy-modified conjugated diene polymer described above.

The xanthogen compound is used by being combined with a vulcanizing agent described below, and thus, is capable of functioning as a vulcanization promoter. That is, in a case where the medical balloon is molded by compounding the vulcanizing agent with the latex composition, and by vulcanizing the carboxy-modified conjugated diene polymer in the latex composition with the vulcanizing agent, the xanthogen compound functions as the vulcanization promoter. In addition, the xanthogen compound functions as the vulcanization promoter with respect to the latex composition with which the vulcanizing agent is compounded, is vulcanized, and then, is decomposed to alcohol, carbon disulfide, and the like by heat to be applied during the vulcanization. For example, the xanthogen compound is decomposed to alcohol, carbon disulfide, and the like by heat to be applied at the time of producing the medical balloon (heat of approximately 100 to 130°C at the time of vulcanizing the carboxy-modified conjugated diene polymer), and a component generated by the decomposition (alcohol, carbon disulfide, and the like) is volatilized. Accordngly, the amount of residual xanthogen compound in the medical balloon to be obtained can be reduced. According to this embodiment, the amount of residual xanthogen compound in the medical balloon to be obtained can be reduced by using the xanthogen compound as the vulcanization promoter without using the vulcanization promoter (for example, a dithiocarbamate-based vulcanization promoter, a thiazole-based vulcanization promoter, and the like) that causes the occurrence of the symptom of delayed-type allergy (Type IV), in the related art, and thus, in the medical balloon to be obtained, the occurrence of the symptom of delayed-type allergy (Type IV) can be suppressed. In addition, the medical balloon to be obtained can be excellent in strength by compounding the xanthogen compound with the latex composition, as the vulcanization promoter. In addition, in the latex composition used in this embodiment, the latex of the carboxy-modified conjugated diene polymer using synthetic rubber of the conjugated diene polymer is used, and thus, in the medical balloon to be obtained, the occurrence of the symptom of immediate-type allergy (Type I) that is caused by protein contained in natural rubber can also be suppressed.

The xanthogen compound is not particularly limited, and examples thereof include a xanthic acid, a xanthate, a xanthogen disulfide (a compound in which two xanthic acids are bonded through a sulfur atom or the like), and a xanthogen polysulfide (a compound three or more xanthic acids are bonded through a sulfur atom or the like).

The xanthate may have a xanthic acid structure, but is not particularly limited, and examples thereof include a compound represented by General Formula (ROC(=S)S)x-Z (Here, R is straight-chain or branched-chain hydrocarbon, Z is a metal atom. x is a number coincident with an atomic valence of Z, and is generally 1 to 4, is preferably 2 to 4, and is particularly preferably 2.).

The xanthate represented by General Formula (ROC(=S)S)x-Z described above is not particularly limited, and examples thereof include zinc methyl xanthate, zinc ethyl xanthate, zinc propyl xanthate, zinc isopropyl xanthate, zinc butyl xanthate, zinc pentyl xanthate, zinc hexyl xanthate, zinc heptyl xanthate, zinc octyl xanthate, zinc 2-ethyl hexyl xanthate, zinc decyl xanthate, zinc dodecyl xanthate, potassium methyl xanthate, potassium ethyl xanthate, potassium propyl xanthate, potassium isopropyl xanthate, potassium butyl xanthate, potassium pentyl xanthate, potassium hexyl xanthate, potassium heptyl xanthate, potassium octyl xanthate, potassium 2-ethyl hexyl xanthate, potassium decyl xanthate, potassium dodecyl xanthate, sodium methyl xanthate, sodium ethyl xanthate, sodium propyl xanthate, sodium isopropyl xanthate, sodium butyl xanthate, sodium pentyl xanthate, sodium hexyl xanthate, sodium heptyl xanthate, sodium octyl xanthate, sodium 2-ethyl hexyl xanthate, sodium decyl xanthate, and sodium dodecyl xanthate. Among them, an alkyl xanthate in which x in General Formula (ROC(=S)S)x-Z described above is greater than or equal to 2 is preferable, and among them, zinc alkyl xanthates are preferably used. In addition, an alkyl xanthate in which R in General Formula (ROC(=S)S)x-Z described above is an isopropyl group or a butyl group is preferably used. Among them, zinc isopropyl xanthate or zinc butyl xanthate is particularly preferably used. Only one type of such xanthates may be used, or a plurality of types thereof may be used together.

The xanthogen disulfide is the compound in which two xanthic acids are bonded through a sulfur atom or the like, but is not particularly limited, and examples thereof include dimethyl xanthogen disulfide, diethyl xanthogen disulfide, diisopropyl xanthogen disulfide, dibutyl xanthogen disulfide, dimethyl xanthogen polysulfide, diethyl xanthogen polysulfide, diisopropyl xanthogen polysulfide, and dibutyl xanthogen polysulfide, and among them, diisopropyl xanthogen disulfide and dibutyl xanthogen disulfide are preferable.

The xanthogen polysulfide is the compound in which three of more xanthic acids are bonded through a sulfur atom or the like, and examples thereof include a xanthogen trisulfide in which three xanthic acids are bonded through sulfur, a xanthogen tetrasulfide in which four xanthic acids are bonded through sulfur, and a xanthogen pentasulfide in which five xanthic acids are bonded through sulfur.

Note that, only one type of such xanthogen compounds may be contained in the latex composition, or two or more types thereof may be contained in the latex composition. For example, in a case where a xanthic acid is compounded with the latex composition, a part of the compounded xanthic acid exists in the form of a xanthate, and thus, two or more

types of xanthogen compounds may be contained in the latex composition. Alternatively, in a case where a part of the xanthic acid compounded with the latex composition contains sulfur as a vulcanizing agent or the like in the latex composition, the xanthic acid compounded with the latex composition may exist in the form of a xanthogen disulfide or a xanthogen polysulfide by the action of sulfur. Similarly, even in a case where a xanthate, a xanthogen disulfide, or a xanthogen polysulfide is compounded with the latex composition, each of the xanthic acid, the xanthogen disulfide, and the xanthogen polysulfide may exist in the form of any one of a xanthic acid, a xanthate, a xanthogen disulfide, a xanthogen polysulfide.

In a case where a content ratio of the xanthogen compound in the latex composition (in a case where a plurality of xanthogen compounds are contained in the latex composition, the total content ratio) is preferably 0.01 to 10 parts by mass, is more preferably 0.1 to 7 parts by mass, and is even more preferably 0.5 to 5 parts by mass, with respect to 100 parts by mass of the carboxy-modified conjugated diene polymer contained in the latex. By setting the content ratio of the xanthogen compound to be in the range described above, in the medical balloon to be obtained, the strength can be further improved while the occurrence of the symptom of delayed-type allergy (Type IV) is suppressed.

Note that, it is preferable that a compound used in the related art as a vulcanization promoter, specifically, a vulcanization promoter containing sulfur that causes the occurrence of the symptom of delayed-type allergy (Type IV) (for example, a dithiocarbamate-based vulcanization promoter, a thiazole-based vulcanization promoter, and the like), which remains in the medical balloon to be obtained after functioning as the vulcanization promoter, is not substantially contained in the latex composition, in addition to the xanthogen compound.

A method for compounding the xanthogen compound with the latex composition may be a method for setting the carboxy-modified conjugated diene polymer and the xanthogen compound to be finally in a state of being mixed in the latex composition, but is not particularly limited, and examples thereof include a method for compounding the xanthogen compound with the latex of the carboxy-modified conjugated diene polymer after obtaining the latex of the carboxy-modified conjugated diene polymer described above, and a method for obtaining the latex of the carboxy-modified conjugated diene polymer with which the xanthogen compound is compounded by compounding in advance the xanthogen compound with a solution or a fine suspension liquid of a carboxy-modified conjugated diene polymer that is dissolved or finely dispersed in an organic solvent, and then, by emulsifying the solution or the fine suspension liquid of the carboxy-modified conjugated diene polymer with which the xanthogen compound is compounded, in water, and as necessary, by removing the organic solvent. Among them, the method for compounding the xanthogen compound with the latex of the carboxy-modified conjugated diene polymer after obtaining the latex of the carboxy-modified conjugated diene polymer is preferable from the viewpoint of easily dissolving the xanthogen compound, and of more easily compounding the xanthogen compound.

## Vulcanizing Agent

The latex composition that can be used as the material of the medical balloon contains a vulcanizing agent, in addition to the carboxy-modified conjugated diene polymer and the xanthogen compound described above. It is preferable to use a vulcanizing agent containing a sulfur-based vulcanizing agent at least as a part thereof, as the vulcanizing agent.

The sulfur-based vulcanizing agent is not particularly limited, and examples thereof include sulfur such as powdered sulfur, sublimed sulfur, precipitated sulfur, colloidal sulfur, surface-treated sulfur, and insoluble sulfur; and a sulfur-containing compound such as sulfur chloride, sulfur dichloride, morpholine·disulfide, alkyl phenol disulfide, caprolactam disulfide (N,N'-dithio-bis(hexahydro-2H-azepinone-2)), phosphorus-containing polysulfide, macromolecular polysulfide, and 2-(4'-morpholinodithio)benzothiazole. Among them, sulfur can be preferably used. Only one type of the sulfur-based vulcanizing agents can be used, or two or more types thereof can be used by being combined.

The content of the sulfur-based vulcanizing agent is not particularly limited, and is preferably 0.1 to 10 parts by mass, and is more preferably 0.2 to 3 parts by mass, with respect to 100 parts by mass of the carboxy-modified conjugated diene polymer contained in the latex composition. By setting the content of the sulfur-based vulcanizing agent to be in the range described above, in the medical balloon to be obtained, the strength can be further increased while the occurrence of the symptom of delayed-type allergy (Type IV) is suppressed.

## Activating Agent

The latex composition that can be used as the material of the medical balloon may contain an activating agent, in addition to the latex of the carboxy-modified conjugated diene polymer, the vulcanizing agent, and the xanthogen compound, described above.

When the carboxy-modified conjugated diene polymer in the latex composition is vulcanized with the sulfur-based vulcanizing agent to be the medical balloon, by using the latex composition to be obtained by compounding the activating agent with the latex composition, the activating agent functions as a vulcanization promoter along with the xanthogen compound described above, and the activating agent itself functions as a cross-linking agent for cross-linking

the carboxyl groups of the carboxy-modified conjugated diene polymer, and thus, the strength of the medical balloon to be obtained is further improved.

The activating agent is not particularly limited, and it is preferable to use a metal compound from the viewpoint of further improving the strength of the medical balloon to be obtained. The metal compound is not particularly limited, and examples thereof include a metal oxide, and a typical metal compound other than an oxide. A metal configuring the metal compound is not particularly limited, and a typical metal (at least one type of element selected from the group consisting of a group I element, a group II element, a group XII element, a group XIII element, a group XIV element, a group XV element, a group XVI element, a group XVII element, and a group XVIII element) is preferable, a group II element, a group XII element, a group XIII element, and a group XIV element are more preferable, zinc, magnesium, calcium, aluminum, and lead are even more preferable, zinc, magnesium, and calcium are particularly preferable, and zinc is most preferable, as the metal. Only one type of such metal compounds may be used, or a plurality of types thereof may be used together.

The metal oxide is not particularly limited, and zinc oxide, magnesium oxide, titanium oxide, calcium oxide, lead oxide, iron oxide, copper oxide, tin oxide, nickel oxide, chromium oxide, cobalt oxide, and aluminum oxide are preferable, zinc oxide is more preferable, from the viewpoint of further improving the strength of the medical balloon to be obtained. Only one type of such metal oxides may be used, or a plurality of types thereof may be used together.

The typical metal compound other than the oxide is a compound containing the typical metal described above, and may be any compound other than an oxide, but is not particularly limited, and a metal compound containing at least one carbon atom is preferable from the viewpoint of further improving the strength of the medical balloon to be obtained, a carbonate, a hydrogen carbonate, a hydroxide, and an organic metal compound are preferable, and a carbonate, a hydrogen carbonate, and an organic metal compound are more preferable, from the viewpoint of further improving the strength of the medical balloon to be obtained. Among them, an inorganic salt such as a carbonate and a hydrogen carbonate is particularly preferable from the viewpoint of excellent stability of compound itself and excellent availability. Only one type of such metal compounds may be used, or a plurality of types thereof may be used together.

A content ratio of the activating agent in the latex composition is preferably 0.01 to 10 parts by mass, is more preferably 0.1 to 5 parts by mass, and is even more preferably 1 to 3 parts by mass, with respect to 100 parts by mass of the carboxy-modified conjugated diene polymer contained in the latex composition. By setting the content ratio of the activating agent to be in the range described above, the strength of the medical balloon to be obtained can be further improved.

A method for compounding the activating agent with the latex composition may be a method for setting the latex of the carboxy-modified conjugated diene polymer and the activating agent to be finally in a state of being mixed, but is not particularly limited, and examples thereof include a method for compounding the activating agent with the latex of the carboxy-modified conjugated diene polymer after obtaining the latex of the carboxy-modified conjugated diene polymer.

## Vulcanization Promoter

The latex composition that can be used as the material of the medical balloon may contain the latex of the carboxy-modified conjugated diene polymer, the vulcanizing agent, and the xanthogen compound, described above, and may further contain a vulcanization promoter insofar as in the medical balloon to be obtained, the occurrence of the symptom of delayed-type allergy (Type IV) can be suppressed.

Vulcanization promoters that are generally used as the vulcanization promoter of the conjugated diene polymer can be used as the vulcanization promoter, and examples of the vulcanization promoter include dithiocarbamic acids such as a diethyl dithiocarbamic acid, a dibutyl dithiocarbamic acid, a di-2-ethyl hexyl dithiocarbamic acid, a dicyclohexyl dithiocarbamic acid, a diphenyl dithiocarbamic acid, and a dibenzyl dithiocarbamic acid, and zinc salts thereof; 2-mercaptobenzothiazole, zinc 2-mercaptobenzothiazole, 2-mercaptothiazoline, dibenzothiazyl·disulfide, 2-(2,4-dinitrophenyl thio)benzothiazole, 2-(N,N-diethyl thio·carbaylthio)benzothiazole, 2-(2,6-dimethyl-4-morpholinothio)benzothiazole, 2-(4'-morpholinodithio)benzothiazole, 4-morpholinyl-2-benzothiazyl·disulfide, and 1,3-bis(2-benzothiazyl·mercaptomethyl)urea, and zinc diethyl dithiocarbamate, zinc dibutyl dithiocarbamate, and zinc 2-mercaptobenzothiazole are preferable. Only one type of such vulcanization promoters can be used, or two or more types thereof can be used by being combined.

## Other Compounding Agents

Further, as necessary, compounding agents such as an age inhibitor; a dispersant; a strengthening agent such as carbon black, silica, and talc; a filler such as calcium carbonate and clay; an ultraviolet absorber; and a plasticizer can be compounded with the latex composition that can be used as the material of the medical balloon.

Examples of the age inhibitor include a phenol-based age inhibitor not containing a sulfur atom, such as 2,6-di-4-methyl phenol, 2,6-di-t-butyl phenol, butyl hydroxyanisole, 2,6-di-t-butyl-α-dimethyl amino-p-cresol, octadecyl-3-(3,5-di-t-butyl-4-hydroxyphenyl) propionate, styrenated phenol, 2,2'-methylene-bis(6-α-methylbenzyl-p-cresol), 4,4'-methylene bis(2,6-di-t-butyl phenol), 2,2'-methylene-bis(4-methyl-6-t-butyl phenol), alkylated bisphenol, and a butylated reaction product of p-cresol and dicyclopentadiene; a thiobisphenol-based age inhibitor such as 2,2'-thiobis-(4-methyl-6-t-butyl phenol), 4,4'-thiobis-(6-t-butyl-o-cresol), and 2,6-di-t-butyl-4-(4,6-bis(octyl thio)-1,3,5-triazin-2-yl amino)phenol; a phosphorous ester-based age inhibitor such as tris(nonyl phenyl) phosphite, diphenyl isodecyl phosphite, and tetraphenyl dipropylene glycol·diphosphate; a sulfur ester-based age inhibitor such as dilauryl thiodipropionate; an amine-based age inhibitor such as phenyl-α-naphthyl amine, phenyl-P-naphthyl amine, p-(p-toluene sulfonyl amide)-diphenyl amine, 4,4'-(α,α-dimethyl benzyl) diphenyl amine, N,N-diphenyl-p-phenylene diamine, N-isopropyl-N'-phenyl-p-phenylene diamine, and a butyl aldehyde-aniline condensate; a quinoline-based age inhibitor such as 6-ethoxy-2,2,4-trimethyl-1,2-dihydroquinoline; and a hydroquinone-based age inhibitor such as 2,5-di-(t-amyl) hydroquinone. Only one type of such age inhibitors may be used, or two or more types thereof can be used together.

The content of the age inhibitor is preferably 0.05 to 10 parts by mass, and is more preferably 0.1 to 5 parts by mass, with respect to 100 parts by mass of the carboxy-modified conjugated diene polymer.

A method for mixing various compounding agents in the latex composition is not particularly limited, and examples thereof include a method for mixing various compounding agents to be compounded with the latex composition, as necessary, by using a dispersion machine such as a ball mill, a kneader, and a disper, after obtaining the latex composition containing the latex of the carboxy-modified conjugated diene polymer, the vulcanizing agent, and the xanthogen compound, as described above, and a method for preparing an aqueous dispersion liquid of a compounded component other than the latex of the carboxy-modified conjugated diene polymer by using the dispersion machine described above, and then, for mixing the aqueous dispersion liquid in the latex of the carboxy-modified conjugated diene polymer.

Note that, a solid content concentration of the latex composition is preferably 10 to 60 weight%, and is more preferably 10 to 55 weight%.

Aging

It is preferable that the latex composition that can be used as the material of the medical balloon is aged (pre-cross-linked) before being provided to molding, from the viewpoint of further increasing the mechanical properties of the medical balloon to be obtained.

An aging temperature may be 5 to 60°C, is preferably 10 to 50°C, and is more preferably 10 to 45°C. In a case where the aging temperature is excessively low, an effect for increasing the mechanical properties of the medical balloon, which is the object for performing the aging, may be insufficient. On the other hand, in a case where the aging temperature is excessively high, the strength of the medical balloon to be obtained may decrease.

An aging time also depends on the temperature, and may be half a day (12 hours) to 14 days, is preferably half a day (12 hours) to 10 days, and is more preferably half a day (12 hours) to 7 days. In a case where the aging time is excessively short, the effect for increasing the mechanical properties of the medical balloon, which is the object for performing the aging, may be insufficient. On the other hand, in a case where the aging time is excessively long, the strength of the medical balloon to be obtained may decrease.

The pH of the latex composition after the aging is preferably greater than or equal to 6 and less than 10, and is more preferably 6 to 9. By setting the pH of the latex composition after the aging to be in the range described above, the strength of the medical balloon to be obtained can be further improved.

Note that, the xanthogen compound is contained in the latex composition, and thus, the pH of the latex composition tends to decrease by the aging. That is, the pH of the latex composition after the aging tends to be lower than the pH of the latex composition before the aging. Here, when the pH of the latex composition varies due to the aging, it is preferable that the pH of the latex composition after the aging remains in the range described above, but in a case where the pH of the latex composition after the aging is not in the range described above, a pH adjuster may be added to the latex composition after the aging, and thus, the pH may be adjusted to be in the range described above. In addition, even in a case where the pH of the latex composition after the aging is in the range described above, the pH adjuster may be added to the latex composition after the aging, in order to make the pH more suitable.

The pH adjuster to be added to the latex composition after the aging is not particularly limited, and examples thereof include a hydroxide of an alkali metal such as sodium hydroxide and potassium hydroxide; a carbonate of an alkali metal such as sodium carbonate and potassium carbonate; a hydrogen carbonate of an alkali metal such as sodium hydrogen carbonate; ammonia; and an organic amine compound such as trimethyl amine and triethanol amine, and a hydroxide of an alkali metal, ammonia, or an organic amine compound is preferable. Only one type of such pH adjusters can be used, or two or more types thereof can be used together.

[Balloon Molding Step]

A molding method for a medical balloon is not particularly limited insofar as a composition containing a carboxy-modified conjugated diene polymer, a vulcanizing agent, and a xanthogen compound is molded into the shape of a balloon, and it is preferable that the method includes a dip molding step that is a step of setting the composition to be a latex composition in the form of latex, and of dipping a balloon-shaped mold in the latex composition. The balloon-shaped mold is dipped in the latex composition, and the latex composition is attached to the surface of the mold, and then, the mold is lifted from the latex composition, and after that, the latex composition deposited on the surface of the mold is dried, and thus, a target medical balloon can be molded.

In the dip molding step, for example, a mold having a shape as illustrated in Fig. 1, which is formed of a metal such as stainless steel, can be used as the mold (the balloon mold) used in the molding of the medical balloon. Such a mold 1 generally includes a columnar portion 2 for forming a pair of cylindrical joint portions (a distal end side joint portion and a proximal end side joint portion) for allowing a distal end portion of a catheter tube of a balloon catheter, which is inserted into the body, to pass therethrough to be closely attached and joined to the surface of the catheter tube, and an oval spherical portion 3 for forming an inflation portion (a portion between the distal end side joint portion and the proximal end side joint portion) that is inflated by being filled with a fluid (for example, the air) to be supplied through the inner cavity (the lumen) of the catheter tube. Note that, the shape of the mold used in the molding of the medical balloon is not particularly limited insofar as a molding body to be obtained can be used as the medical balloon, and for example, a simple columnar body or the like may be used.

Note that, the mold before being dipped in the latex composition may be preheated. In addition, as necessary, a coagulant can be used before the mold is dipped in the latex composition or after the mold is lifted from the latex composition.

Specific examples of a use method of the coagulant include a method for attaching the coagulant to the mold by dipping the mold before being dipped in the latex composition in a coagulant solution (an anode adhesion dipping method), and a method for dipping the mold on which the latex composition is deposited in the coagulant solution (a Teague adhesion dipping method), and the anode adhesion dipping method is preferable from the viewpoint of obtaining a medical balloon with small thickness unevenness.

A specific example of the coagulant is a soluble polyvalent metal salt such as a halogenated metal such as barium chloride, calcium chloride, magnesium chloride, zinc chloride, and aluminum chloride; a nitrate such as barium nitrate, calcium nitrate, and zinc nitrate; an acetate such as barium acetate, calcium acetate, and zinc acetate; and a sulfate such as calcium sulfate, magnesium sulfate, and aluminum sulfate. Among them, a calcium salt is preferable, and calcium nitrate is more preferable. Only one type of such soluble polyvalent metal salts can be used, or two or more types thereof can also be used together.

In general, the coagulant can be used as a solution of water, alcohol, or a mixture thereof, and is preferably used in the state of an aqueous solution. Such an aqueous solution may further contain a soluble organic solvent such as methanol and ethanol, or a nonionic surfactant. The concentration of the coagulant is different in accordance with the type of soluble polyvalent metal salt, and is preferably 5 to 50 weight%, and is more preferably 10 to 30 weight%.

In general, heating is performed after the mold is lifted from the latex composition, and thus, an attachment formed on the mold is dried. A drying condition may be suitably selected.

Next, a layer of the composition formed on the mold is cross-linked by heating. In general, the layer of the composition can be cross-linked by performing a heating treatment at a temperature of 80 to 150°C, preferably for 10 to 130 minutes. A method using external heating of an infrared ray or heating air or a method using internal heating of a high-frequency wave can be adopted as a heating method. Among them, the external heating of the heating air is preferable. Note that, before the heating treatment is performed, the composition may be dipped in water, preferably hot water of 30 to 70°C, for approximately 1 to 60 minutes, and thus, soluble impurities (for example, an excessive emulsifier, coagulant, or the like) may be removed. A removing operation of the soluble impurities may be performed after the layer of the composition is subjected to the heating treatment, and it is preferable that the removing operation is performed before the heating treatment from the viewpoint of more efficiently removing the soluble impurities.

Then, the medical balloon obtained on the mold as described above is released from the mold, and thus, a target medical balloon is obtained. A method for removing the mold with hands, or a method for removing the mold with a water pressure or a compressed air pressure can be adopted as a mold releasing method. Note that, after the mold releasing, the heating treatment may be further performed at a temperature of 60 to 120°C for 10 to 120 minutes.

The medical balloon to be obtained by the production method of this embodiment is obtained by using the composition described above, and thus, the occurrence of the symptom of not only immediate-type allergy (Type I) but also delayed-type allergy (Type IV) can be suppressed, and the strength is excellent, and therefore, a dimensional change rate of the balloon after inflation and contraction are repeated is small, and durability is also excellent.

Note that, the medical balloon produced by the production method of this embodiment can be widely used to configure various balloon-equipped medical devices including various balloon catheters such as a balloon catheter used

to remove impurities such as calculus (bilestone) generated in the body by using an inflated balloon and a balloon catheter having a function of fixing (anchoring) a catheter tube in a desired position of the lumen in the body with the inflated balloon and various balloon-equipped endoscopes such as a double balloon endoscope.

## EXAMPLES

Hereinafter, the invention will be described in more detail by examples, but the invention is not limited to such examples. Note that, hereinafter, unless otherwise specified, "parts" are based on weight. In addition, various physical property measurements and evaluation tests were performed as follows.

### Solid Content Concentration

On an aluminum plate (Weight: X1), 2 g of a sample was accurately weighed (Weight: X2), and was dried in a hot-air drier of 105°C for 2 hours. Next, cooling was performed in a desiccator, and then, the weight of the aluminum plate was measured (Weight: X3), and thus, a solid content concentration was calculated in accordance with the following calculation expression.

$$\text{Solid Content Concentration (Weight\%)} = \{(X3 - X1)/X2\} \times 100$$

### Modification Rate

A carboxy-modified conjugated diene polymer configuring the latex of the carboxy-modified conjugated diene polymer was measured with $^1$H-NMR, and thus, the number of carboxyl groups in the carboxy-modified conjugated diene polymer was obtained. Next, a modification rate due to a carboxyl group-containing compound was obtained on the basis of the obtained number of carboxyl groups, in accordance with Expression (ii) described below.

$$\text{Modification Rate (Mol\%)} = (X/Y) \times 100 \cdots\text{(ii)}$$

Note that, in the Expression (ii) described above, X indicates the number of carboxyl groups in the carboxy-modified conjugated diene polymer, and Y indicates the total number of structure units of the carboxy-modified conjugated diene polymer ((Weight Average Molecular Weight (Mw) of Carboxy-Modified Conjugated Diene Polymer)/(Average Molecular Weight according to Content Ratio of Each Structure Unit Configuring Carboxy-Modified Conjugated Diene Polymer)).

### Content Ratio of Aggregate

A latex composition was left to still at 30°C for 2 days, and then, a solid content concentration of the latex composition was measured in accordance with the method described above, 100 g of the latex composition was weighed, and then, was filtered with an SUS wire mesh of 200 meshes, of which the weight was known, an aggregate on the wire mesh was subjected to water washing several times, and the latex composition was removed. This was dried at 105°C for 2 hours or longer, and then, the dry weight was measured, and a content ratio (Unit: Weight%) of the aggregate was obtained on the basis of the following expression.

$$\text{Content Ratio (weight\%) of Aggregate} = \{(\alpha - \beta)/(\gamma \times \Delta)\} \times 10{,}000$$

Here, $\alpha$ indicates the weight of the wire mesh after the drying and the dry aggregate, $\beta$ indicates the weight of the wire mesh, $\gamma$ indicates the weight of the latex composition, and $\Delta$ indicates the solid content concentration (Unit: Weight%) of the latex composition.

### Patch Test

A test piece obtained by being cut out from a molded balloon to have a size of 10 × 10 mm was pasted to each arm of ten subjects to be tested. After that, pasted portion was observed after 48 hours, and thus, it was checked whether or not the allergy symptom of delayed-type allergy (Type IV) occurred, and evaluation was performed on the following criteria.

A: No allergy symptom was seen in all of the subjects to be tested.
B: The allergy symptom was seen in a part of the subjects to be tested.

Dimensional Change Rate of Balloon after Repeating Inflation and Contraction

In a state in which the molded balloon was attached to a dedicated jig, the maximum outer diameter of the balloon in the initial state was measured with a laser outer diameter measuring instrument. Next, each 1 ml of the air was injected into the balloon with a syringe, and the balloon was inflated. The air was discharged from the inside of the balloon at a time point when the maximum outer diameter of the balloon exceeded 30 mm, and the balloon was contracted. The maximum outer diameter of the balloon after such an inflation and contraction operation was repeated 20 times was measured, and a dimensional change rate was obtained in accordance with Expression (iii) described below.

$$\text{Dimensional Change Rate (\%) of Balloon} = \{(Y - X)/X\} \times 100 \cdots \text{(iii)}$$

Note that, in Expression (iii) described above, X indicates maximum outer diameter (mm) of the balloon in the initial state, and Y indicates the maximum outer diameter (mm) of the balloon after the inflation and contraction operation was repeated 20 times.

Durability of Balloon

In a state where the molded balloon was attached to the tip end of a catheter tube with an adhesive agent, the balloon was inflated in water by injecting the air into the balloon with the syringe through the inner cavity of the catheter tube until the maximum outer diameter of the balloon reached 30 mm. Next, the balloon was contracted until the maximum outer diameter of the balloon reached 20 mm, and then, the balloon was allowed to pass through a stainless steel plate with a hole having a diameter of 18 mm. Finally, the air was discharged from the inside of the balloon, and the balloon was contracted. Such a set of operations was repeated, the number of times until an air leakage from the balloon (checked by the occurrence of air bubbles) or the burst of the balloon occurred when the balloon was inflated in water was measured, and the number of times was set to an index of the durability of the balloon. Note that, when the air leakage from the balloon or the burst of the balloon was not seen even in a case where a set of operations was repeated 100 times, an evaluation test of the durability of the balloon was ended at the time point.

Production Example 1

Production of Latex of Carboxy-Modified Synthetic Polyisoprene (A-1)

Synthetic polyisoprene having a weight average molecular weight of 1,300,000 (Product Name "NIPOL IR2200L", manufactured by Zeon Corporation, an isoprene homopolymer, a cis bonding unit amount of 98%) was mixed with cyclohexane, and was dissolved by increasing the temperature to 60°C while being stirred, and thus, a cyclohexane solution (a) of synthetic polyisoprene, of which the viscosity measured with a B type viscosimeter was 12,000 mPa·s, was prepared (a solid content concentration of 8 weight%).

On the other hand, 20 parts of sodium rosinate was added to water, and was dissolved by increasing the temperature to 60°C, and thus, an anionic surfactant aqueous solution (b) having a concentration of 1.5 weight% was prepared.

Next, the cyclohexane solution (a) described above and the anionic surfactant aqueous solution (b) described above were mixed such that a weight ratio was 1 : 1.5 by using a mixer (Product Name "Multi-Line Mixer MS26-MMR-5.5L", manufactured by Satake Chemical Equipment Mfg., Ltd.), and then, were mixed and emulsified at the number of rotations of 4100 rpm by using an emulsification device (Product Name "MilderMDN310", manufactured by Pacific Machinery & Engineering Co., Ltd), and thus, an emulsified liquid (c) was obtained. Note that, at this time, the total feed flow rate of the cyclohexane solution (a) and the anionic surfactant aqueous solution (b) was 2,000 kg/hr, a temperature was 60°C, and a back pressure (a gauge pressure) was 0.5 MPa.

Next, the emulsified liquid (c) was heated to 80°C under a reduced pressure of - 0.01 to -0.09 MPa (a gauge pressure), cyclohexane was distilled away, and thus, a water dispersion liquid (d) of synthetic polyisoprene was obtained. At this time, an antifoaming agent (Product Name "SM5515", manufactured by Dow Toray Co.,Ltd.) was continuously added to synthetic polyisoprene in the emulsified liquid (c) such that the amount was 300 weight ppm while being sprayed. Note that, when cyclohexane is distilled away, the emulsified liquid (c) was adjusted to be less than or equal to 70 volume% of the volume of a tank, and was slowly stirred at 60 rpm by using a triple inclined paddle blade as a stirring blade.

Then, after cyclohexane was completely distilled away, the obtained water dispersion liquid (d) of synthetic

polyisoprene was subjected to centrifugal separation at 4,000 to 5,000 G by using a continuous centrifugal separation machine (Product Name "SRG510", manufactured by Alfa Laval AB), and thus, latex (e) of synthetic polyisoprene having a solid content concentration of 56 weight%, as a light liquid, was obtained. Note that, as the condition of the centrifugal separation, a solid content concentration of the water dispersion liquid (d) before the centrifugal separation was 10 weight%, a flow rate in the continuous centrifugal separation was 1300 kg/hr, and a back pressure (a gauge pressure) of a centrifugal separation machine was 1.5 MPa. In the obtained latex (e) of synthetic polyisoprene, a solid content concentration was 60 weight%.

Next, 130 parts of distilled water was added to 100 parts of synthetic polyisoprene in the obtained latex (e) of synthetic polyisoprene, and dilution was performed. Then, 0.8 parts of a sodium salt of a β-naphthalene sulfonate formalin condensate (Product Name "DEMOL T-45", manufactured by Kao Corporation), as a dispersant, with respect to 100 parts of synthetic polyisoprene was diluted with 4 parts of distilled water with respect to 100 parts of synthetic polyisoprene, and was added to the latex (e) of synthetic polyisoprene for 5 minutes. Next, the latex (e) of synthetic polyisoprene to which the dispersant was added was put in a reaction vessel with a stirrer that was subjected to nitrogen substitution, and the temperature was increased to 30°C while stirring was performed. In addition, 3 parts of a methacrylic acid, as the carboxyl group-containing compound, and 16 parts of distilled water were mixed by using another vessel, and thus, a diluted solution of a methacrylic acid was prepared. The diluted solution of a methacrylic acid was added to a reaction vessel that was heated to 30°C, for 30 minutes.

Further, a solution (f) containing 7 parts of distilled water, 0.32 parts of sodium formaldehyde sulfoxylate (Product Name "SFS", manufactured by Mitsubishi Gas Chemical Company, Inc.), and 0.01 parts of ferrous sulfate (Product Name "Frost Fe", manufactured by Chubu Chelest Co., Ltd.) was prepared by using another vessel. The solution (f) was moved into a reaction vessel, and then, 0.5 parts of 1,1,3,3-tetramethyl butyl hydroperoxide (Product Name "Perocta H", manufactured by NOF CORPORATION) was added thereto, and a reaction was performed at 30°C for 1 hour, and thus, latex of carboxy-modified synthetic polyisoprene (A-1) was obtained. Next, carboxy-modified synthetic polyisoprene (A-1) was concentrated with a centrifugal separation machine, and thus, a light liquid having a solid content concentration of 56% was obtained. Then, a modification rate of the obtained latex of carboxy-modified synthetic polyisoprene (A-1) was measured in accordance with the method described above, and the modification rate was 0.5 mol%.

Production Example 2

Production of Latex of Carboxy-Modified Synthetic Polyisoprene (A-2)

Latex of carboxy-modified synthetic polyisoprene (A-2) having a solid content concentration of 55% was obtained as with Production Example 1 except that a use amount of a methacrylic acid was changed to 5 parts from 3 parts. A modification rate of the obtained latex of carboxy-modified synthetic polyisoprene (A-2) was measured in accordance with the method described above, and the modification rate was 1 mol%.

Example 1

Preparation of Latex Composition

First, in a styrene-maleate mono-sec-butyl ester-maleate monomethyl ester polymer (Product Name "Scripset550", manufactured by Hercules Incorporated), 100% of carboxyl groups in the polymer were neutralized by using sodium hydroxide, and thus, an aqueous solution of a sodium salt (a concentration of 10 weight%) was prepared. Then, the aqueous solution of the sodium salt was added to the latex of carboxy-modified synthetic polyisoprene (A-1) obtained in Production Example 1 to be 0.8 parts with respect to 100 parts of carboxy-modified synthetic polyisoprene (A-1), in terms of a solid content, and thus, a mixture was obtained.

Then, 2 parts of zinc isopropyl xanthate, as a xanthogen compound, was added to 100 parts of carboxy-modified synthetic polyisoprene (A-1) in the mixture while the obtained mixture was stirred.

Next, a water dispersion liquid of each compounding agent was added to be 1.5 parts of zinc oxide, as an activating agent, 1.5 parts of sulfur, and 2 parts of an age inhibitor (Product Name "Wingstay L", manufactured by Goodyear Tire and Rubber Company) in terms of a solid content, and thus, a latex composition was obtained. An aqueous solution of potassium hydroxide (a concentration of 5.0 weight%), as a pH adjuster, was added to the obtained latex composition, and thus, pH was adjusted to 8.0 (the pH of the latex composition before aging was set to 8.0).

Then, the obtained latex composition was aged in a constant temperature water tank that was adjusted to 30°C, for 48 hours. The pH of the latex composition after the aging was 7.5.

Production of Medical Balloon

As illustrated in Fig. 1, the stainless steel balloon mold 1 was used in which the diameter of the columnar portion 2 was 2.5 mm, the diameter of the oval spherical portion 3 was 5.0 mm, and the length of the oval spherical portion 3 was 7 mm. First, the balloon mold was washed, and was preheated in an oven of 70°C, and then, was dipped in an aqueous solution of a coagulant containing 18 weight% of calcium nitrate and 0.05 weight% of polyoxyethylene lauryl ether (Product Name "EMULGEN 109P", manufactured by Kao Corporation) for 5 seconds, and was extracted from the aqueous solution of the coagulant. Next, the balloon mold was dried in an oven of 70°C for 30 minutes or longer, and thus, the coagulant was attached to the balloon mold, and the balloon mold was covered with the coagulant.

After that, the balloon mold covered with the coagulant was extracted from the oven, and was dipped in the latex composition after the aging, described above, for 15 seconds. Next, the balloon mold was dried with air at a room temperature for 10 minutes, and then, was dipped in hot water of 60°C for 5 minutes such that soluble impurities were eluted, and thus, a dip molding layer was formed on the balloon mold. After that, the dip molding layer formed on the balloon mold was cross-linked by being heated with an oven in a condition of a temperature of 120°C and 30 minutes, and then, was cooled to a room temperature, and was peeled out from the balloon mold after talc was dispersed, and a molding body that was obtained was cut such that an oval spherical portion was positioned in the center and the total length was 15 mm, and thus, a target medical balloon having a film thickness of 0.25 mm was obtained. Then, in the obtained medical balloon, a dimensional change rate and durability were respectively evaluated in accordance with the method described above. Results are shown in Table 1. Note that, in Table 1, a compounded amount of the dispersant was described as an amount with respect to 100 parts of synthetic polyisoprene before being subjected to carboxy modification.

Example 2

A medical balloon was produced as with Example 1 except that the latex of carboxy-modified synthetic polyisoprene (A-2) (100 parts in terms of carboxy-modified synthetic polyisoprene (A-2)) obtained in Production Example 2 was used instead of the latex of carboxy-modified synthetic polyisoprene (A-1) obtained in Production Example 1, at the time of preparing the latex composition, and similarly, evaluation was performed. Results are shown in Table 1.

Comparative Example 1

A medical balloon was produced as with Example 1 except that the latex (e) of synthetic polyisoprene (100 parts in terms of synthetic polyisoprene) obtained in Production Example 1 was directly used instead of the latex of carboxy-modified synthetic polyisoprene (A-1) obtained in Production Example 1, at the time of preparing the latex composition, and similarly, evaluation was performed. Results are shown in Table 1.

Comparative Example 2

A medical balloon was produced as with Example 1 except that a total of 1.5 parts of a dithiocarbamate-based vulcanization promoter and a thiazole-based vulcanization promoter (0.3 parts of zinc diethyl dithiocarbamate, 0.5 parts of zinc dibutyl dithiocarbamate, and 0.7 parts of zinc 2-mercaptobenzothiazole) was added instead of 2 parts of zinc isopropyl xanthate, as the xanthogen compound, at the time of preparing the latex composition, and similarly, evaluation was performed. Note that, the pH of the latex composition before the aging was 8.0 as with Example 1, and the pH of the latex composition after the obtained latex composition was aged in a constant temperature water tank that was adjusted to 30°C, for 48 hours, remained 8.0 without a change. Results are shown in Table 1.

[Table 1]

Table 1

| | | Example | | Comparative Example | |
|---|---|---|---|---|---|
| | | 1 | 2 | 1 | 2 |
| **Compounding** | | | | | |
| Carboxy-modified synthetic polyisoprene (A-1) (modification rate of 0.5 mol%) | (parts) | 100 | - | - | 100 |
| Carboxy-modified synthetic polyisoprene (A-2) (modification rate of 1 mol%) | (parts) | - | 100 | - | - |
| Synthetic polyisoprene | (parts) | - | - | 100 | - |
| Zinc isopropyl xanthate | (parts) | 2 | 2 | 2 | - |
| Dithiocarbamate-based vulcanization promoter and thiazole-based vulcanization promoter | (parts) | - | - | - | 1.5 |
| Zinc oxide | (parts) | 1.5 | 1.5 | 1.5 | 1.5 |
| Sulfur | (parts) | 1.5 | 1.5 | 1.5 | 1.5 |
| Dispersant | (parts) | 0.8 | 0.8 | 0.8 | 0.8 |
| **Evaluation** | | | | | |
| Content ratio of aggregate in latex composition | (weight%) | 0.02 | 0.015 | 0.3 | 0.02 |
| Patch test | | A | A | A | B |
| Dimensional change rate of balloon after repeating inflation and contraction | (%) | 5 | 10 | 10 | 20 |
| Durability of balloon | (times) | > 100 | > 100 | 30 | 50 |

From Table 1, the latex composition containing the latex of the carboxy-modified conjugated diene polymer, the vulcanizing agent, and the xanthogen compound was excellent in stability since the occurrence of the aggregate was suppressed, and the medical balloon produced by using the latex composition was less likely to cause the allergy symptom even in the case of being in contact with the human body, had a small dimensional change rate of the balloon after the inflation and contraction were repeated, and was excellent in the durability of the balloon (Examples 1 and 2).

On the other hand, the latex composition obtained by using the latex of synthetic polyisoprene that was not subjected to the carboxy modification, instead of the latex of the carboxy-modified conjugated diene polymer, had a large occurrence amount of the aggregate, and was poor in the stability, and the medical balloon produced by using the latex composition was poor in the durability of the balloon (Comparative Example 1).

In addition, in a case where the dithiocarbamate-based vulcanization promoter and the thiazole-based vulcanization promoter were compounded instead of the xanthogen compound, the medical balloon produced by using the obtained latex composition was likely to cause the allergy symptom in the case of being in contact with the human body, had a large balloon dimensional change rate after the inflation and contraction were repeated, and was poor in the durability of the balloon (Comparative Example 2).

EXPLANATIONS OF LETTERS OR NUMERALS

1 MOLD
2 CYLINDRICAL PORTION
3 OVAL SPHERICAL PORTION

## Claims

**1.** A production method for a medical balloon, comprising:

molding a composition containing a carboxy-modified conjugated diene polymer, a vulcanizing agent, and a xanthogen compound into a shape of a balloon.

**2.** The production method for a medical balloon according to claim 1,
wherein the vulcanizing agent includes a sulfur-based vulcanizing agent.

**3.** The production method for a medical balloon according to claim 1 or 2,
wherein the composition further contains a metal oxide or a typical metal compound other than an oxide, as an activating agent.

**4.** The production method for a medical balloon according to any one of claims 1 to 3,
wherein molding the composition into the shape of a balloon comprises dipping a balloon-shaped mold in the composition of a form of latex.

Figure 1

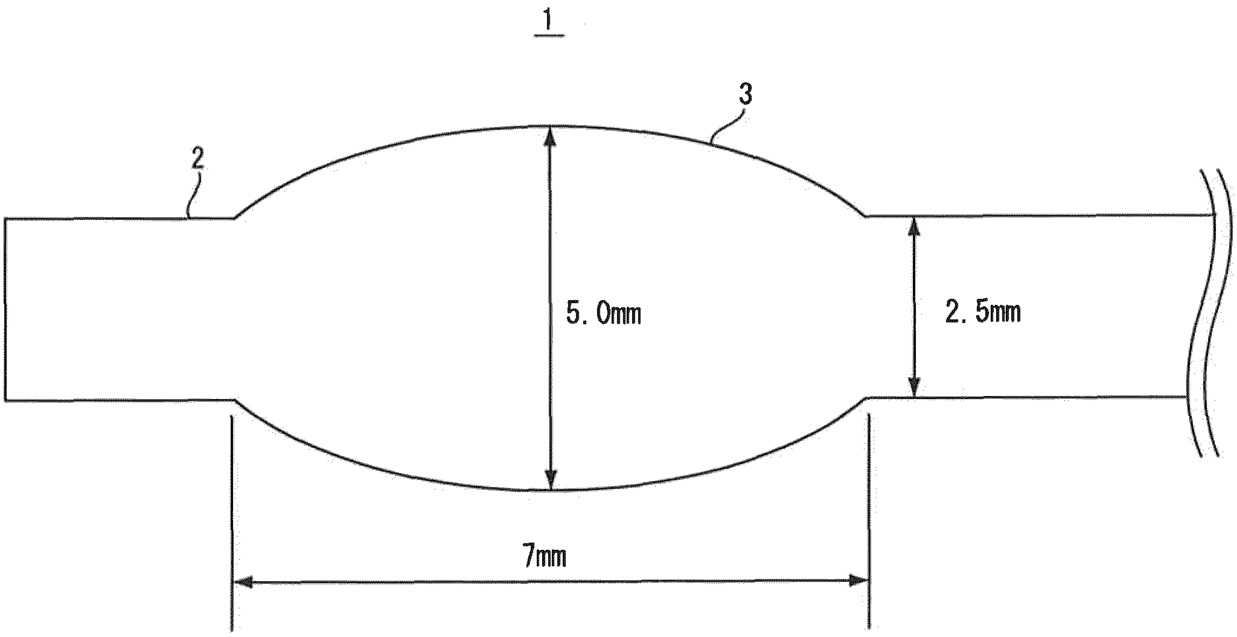

## INTERNATIONAL SEARCH REPORT

International application No. PCT/JP2019/032273

A. CLASSIFICATION OF SUBJECT MATTER
Int.Cl. A61M25/10(2013.01)i, C08C19/28(2006.01)i, C08K5/39(2006.01)i, C08L15/00(2006.01)i

According to International Patent Classification (IPC) or to both national classification and IPC

B. FIELDS SEARCHED

Minimum documentation searched (classification system followed by classification symbols)
Int.Cl. A61M25/10, C08C19/28, C08K5/39, C08L15/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2014/129547 A1 (ZEON CORPORATION) 28 August 2014, paragraphs [0016], [0038], [0075]-[0087], [0097] & US 2015/0376322 A1, paragraphs [0023], [0024], [0050], [0095]-[0110], [0123] & EP 2960293 A1 | 1-4 |
| Y | JP 2013-534555 A (ALLEGIANCE CORPORATION) 05 September 2013, paragraphs [0003]-[0030], [0038], [0039], [0045] & US 2012/0021155 A1, paragraphs [0005]-[0030], [0038], [0039], [0045] & WO 2011/163662 A1 & CN 103025514 A & KR 10-2013-0141429 A | 1-4 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17.09.2019 | 01.10.2019 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/032273

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-229129 A (ZEON CORPORATION) 13 September 2007, paragraph [0028]<br>(Family: none) | 1-4 |
| Y | JP 2016-525164 A (SKINPROTECT CORPORATION SDN BHD) 22 August 2016, paragraph [0174]<br>& US 2016/0159992 A1 paragraph [0184] & WO 2015/006806 A1 & CN 105492179 A | 1-4 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014129547 A1